# EUROPEAN PATENT APPLICATION

(11) **EP 4 428 133 A1**
(43) Date of publication of application: **11.09.2024**
(21) Application number: 23160534.6
(22) Date of filing: 07.03.2023
(51) Int. Cl.: C07D 471/12, C07D 487/04, A61P 35/00, A61K 31/5025

(54) **BIFUNCTIONAL PROTEIN TARGETING CHIMERAS (PROTACS) COMPRISING ARYLIDENE-INDOLINONE SCAFFOLD AS DCAF11 LIGAND AND USE THEREOF**

(71) Applicant: Max-Planck-Gesellschaft zur Förderung der Wissenschaften e.V., 80539 München (DE); CeMM - Forschungszentrum für Molekulare Medizin GmbH, 1090 Wien (AT)
(72) Inventor: The designation of the inventor has not yet been filed
(74) Representative: Arth, Hans-Lothar

(57) **Abstract**

The present invention relates to compounds of the formula (I) as bifunctional protein targeting chimeras (PROTACs), pharmaceutically acceptable salts thereof, and pharmaceutical compositions thereof. These compounds, pharmaceutically acceptable salts, and pharmaceutical compositions thereof are useful in the treatment or prophylaxis of a cancer, an autoimmune disease, or an inflammatory disease, in paticular, associated with and/or caused by PDEdelta (PDEδ) / K-Ras, or B lymphoid *kinase* (BLK) /Bruton's tyrosine kinase (BTK).

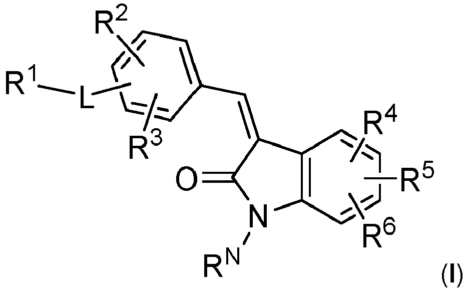

## Description

The present invention relates to compounds of the formula (I) as bifunctional protein targeting chimeras (PROTACs), pharmaceutically acceptable salts thereof, and pharmaceutical compositions thereof. These compounds, pharmaceutically acceptable salts, and pharmaceutical compositions thereof are useful in the treatment or prophylaxis of a cancer, an autoimmune disease, or an inflammatory disease, in paticular, associated with and/or caused by PDEdelta(PDEδ)/K-Ras kinase, or B lymphoid *kinase*(BLK)/Bruton's tyrosine kinase (BTK).

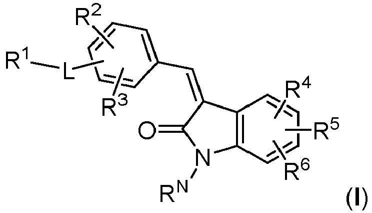

### Background of the invention

Targeted protein degradation (TPD) has emerged as a new paradigm for modulation of protein activity in chemical biology and drug discovery. A viable strategy for TPD employs protein targeting chimeras, in which small molecule protein ligands are linked to a ligand of an E3 ligase to yield bifunctional modalities that induce degradation of the protein of interest by the ubiquitin-proteasome system (UPS) [Bekes, M., Langley, D. R. & Crews, C. M. PROTAC targeted protein degraders: the past is prologue. Nat. Rev. Drug Discov. 21, 181-200 (2022)]. Recently also bifunctional degrader modalities have been reported that direct the protein of interest to the autophagy-related proteins LC3B or p62, thereby inducing protein degradation by means of macroautophagy (Pei, J. et al. Developing potent LC3-targeting AUTAC tools for protein degradation with selective autophagy. Chem. Commun. 57, 13194-13197 (2021); Ji, C. H. et al. The AUTOTAC chemical biology platform for targeted protein degradation via the autophagy-lysosome system. Nat. Commun. 13, 904 (2022)). In particular, it was reported that an α, β-unsaturated indolinone targets LC3B and thereby induces degradation of huntingtin by macroautophagy (Li, Z. et al. Allele-selective lowering of mutant HTT protein by HTT-LC3 linker compounds. Nature 575, 203-209 (2019)), and that a bifunctional degrader derived from **1** primes the BRD4 protein for autophagic degradation (Pei, J. et al. Developing potent LC3-targeting AUTAC tools for protein degradation with selective autophagy. Chem. Commun. 57, 13194-13197 (2021)).

PROTAC-mediated degradation through the UPS has been applied to the lipoprotein-binding chaperone PDEδ which regulates the cellular distribution, and thereby the activity of the Ras-oncoproteins and other lipidated proteins [Winzker, M. et al. Development of a PDEdelta-targeting PROTACs that impair lipid metabolism. Angew. Chem. Int. Ed. 59, 5595-5601 (2020)]. To mitigate associated limitations, we set out to explore whether effective degradation of PDEδ would be possible by means of macroautophagy, initiated by a bifunctional modality combining ligands of LC3B and PDEδ.

It is object to the present invention to provide novel compounds of the formula (I) as bifunctional protein targeting chimeras (PROTACs), pharmaceutically acceptable salts thereof, which can be used as pharmaceutically active agents, and use thereof especially for prophylaxis and/or treatment of a cancer, an autoimmune disease, or an inflammatory disease, in paticular, associated with and/or caused by PDEdelta(PDE5)/K-Ras kinase, or B lymphoid *kinase*(BLK)/Bruton's tyrosine kinase (BTK), as well as compositions comprising at least one of those compounds and/or pharmaceutically acceptable salts thereof as pharmaceutically active ingredients.

The objective of the present invention is solved by the teaching of the independent claims. Further advantageous features, aspects and details of the invention are evident from the dependent claims, the description, and the examples of the present application.

### Description of the invention

The present invention relates to a compound of the general formula (I) 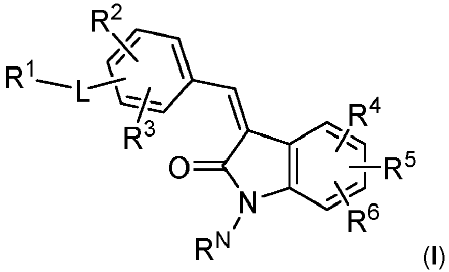 wherein
**L** represents **-L₃-L₂-L₁-;**
**L₁** represents a covalent bond, -O-, -NH-, or -NH-CO-CH₂-O-;
**L₂** represents -(C₂H₄)ₙ-, -CH₂-(C₂H₄)ₙ-, -(C₂H₄O)ₙ-, -(CH₂)ₘ-(C₂H₄O)ₙ-, -(C₂H₄O)ₙ-(CH₂)ₘ-, -(CH₂)ₘ-(OC₂H₄)ₙ-, -(OC₂H₄)ₙ-(CH₂)ₘ-, -(CH₂)ₘ-(C₂H₄O)ₙ-(CH₂)ₒ-, -(CH₂)ₘ-(OC₂H₄)ₙ-(CH₂)ₒ-, or -(CH₂)ₘ-(OC₂H₄)ₙ-O-(CH₂)ₒ-;
**L₃** represents a covalent bond, -O-, -NH-, -NH-CO-, -CO-NH-, -CH₂-CO-NH-, or -NH-CO-NH-;
**n** is an integer selected from 1, 2, 3, 4, 5, 6, 7, 8, 9, and 10;
**m** and **o** are independently of each other an integer selected from 1, 2, 3, and 4;
**R¹** represents 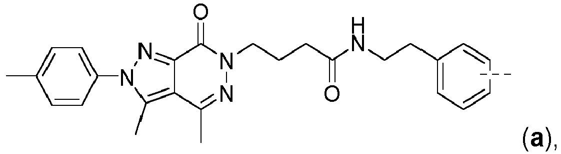 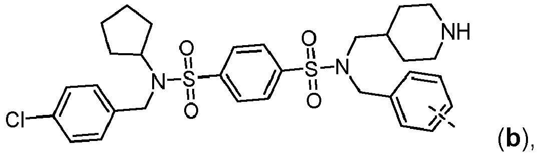 or 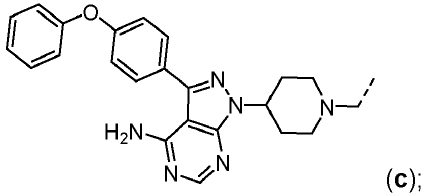
**R²**, **R³**, **R⁴**, **R⁵**, and **R⁶** represent independently of each other -H, -F, -Br, -Cl, -I, -OH, -CN, -NO₂, -CH₃, -C₂H₅, -C₃H₇, -CH(CH₃)₂, -C₄H₉, -CH₂-CH(CH₃)₂, -cyclo-C₃H₅, -cyclo-C₄H₇, -cyclo-C₅H₉, -cyclo-C₆H₁₁, -OCH₃, -OC₂H₅, -OCOCH₃, -OC₃H₇, -O-cyclo-C₃H₅, -OCH(CH₃)₂, -OC(CH₃)₃, -OC₄H₉, -OPh, -OCH₂-Ph, -OCH₂F, -OCHF₂, -OCF₃, -CH₂F, -CHF₂, -CF₃, -CH₂-CH₂F, -CH₂-CHF₂, -CH₂-CF₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)_{2]}, -CONH[C(CH₃)_{3]}, -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, , -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)_{2]}, -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH=CH₂, -CH₂-CH=CH₂, -C(CH₃)=CH₂, -CH=CH-CH₃, -CH=CH-Ph, -C≡CH, -C≡C-CH₃, -CH₂-C≡CH, 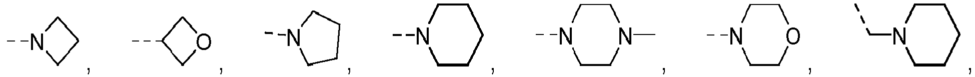 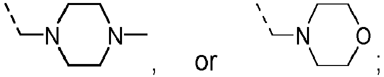
**R**^{N} represents -H, -CH₃, -C₂H₅, -C₃H₇, -CH(CH₃)₂, -C₄H₉, -CH₂-CH(CH₃)₂, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂Ph, -COCH₃, -COCF₃, -COC₂H₅, -COC₃H₇, -COCH(CH₃)₂, -COC₄H₉, -COCH₂-CH(CH₃)₂, -COC(CH₃)₃, -CO-cyclo-C₃H₅, -CO-cyclo-C₄H₇, -CO-cyclo-C₅H₉, -CO-cyclo-C₆H₁₁, -COPh, -COCH₂Ph, -CO₂CH₃, -CO₂CF₃, -CO₂C₂H₅, -CO₂C₃H₇, -CO₂CH(CH₃)₂, -CO₂C₄H₉, -CO₂CH₂-CH(CH₃)₂, -CO₂C(CH₃)₃, -CO₂-cyclo-C₃H₅, -CO₂-cyclo-C₄H₇, -CO₂-cyclo-C₅H₉, -CO₂-cyclo-C₆H₁₁, -CO₂Ph, -CO₂CH₂Ph, -SO₂CH₃, -SO₂CF₃, -SO₂C₂H₅, -SO₂C₃H₇, -SO₂CH(CH₃)₂, -SO₂C₄H₉, -SO₂CH₂-CH(CH₃)₂, -SO₂C(CH₃)₃, -SO₂-cyclo-C₃H₅, -SO₂-cyclo-C₄H₇, -SO₂-cyclo-C₅H₉, -SO₂-cyclo-C₆H₁₁, -SO₂Ph, or -SO₂CH₂Ph;
or an enantiomer, a stereoisomeric form, a mixture of enantiomers, a diastereomer, a mixture of diastereomers, a hydrate, a solvate, a racemate of the above mentioned compounds, or a pharmaceutically acceptable salt thereof.

The term "pharmaceutically acceptable salt" refers to a salt of a compound that does not cause significant irritation to an organism to which it is administered and does not abrogate the biological activity and properties of the compound. The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art.

Preferred embodiments of the present invention relate to the compounds having any one of the formulae (**Ia**) - (**Id**) and (**IIaB)** - (**IId**)**:**

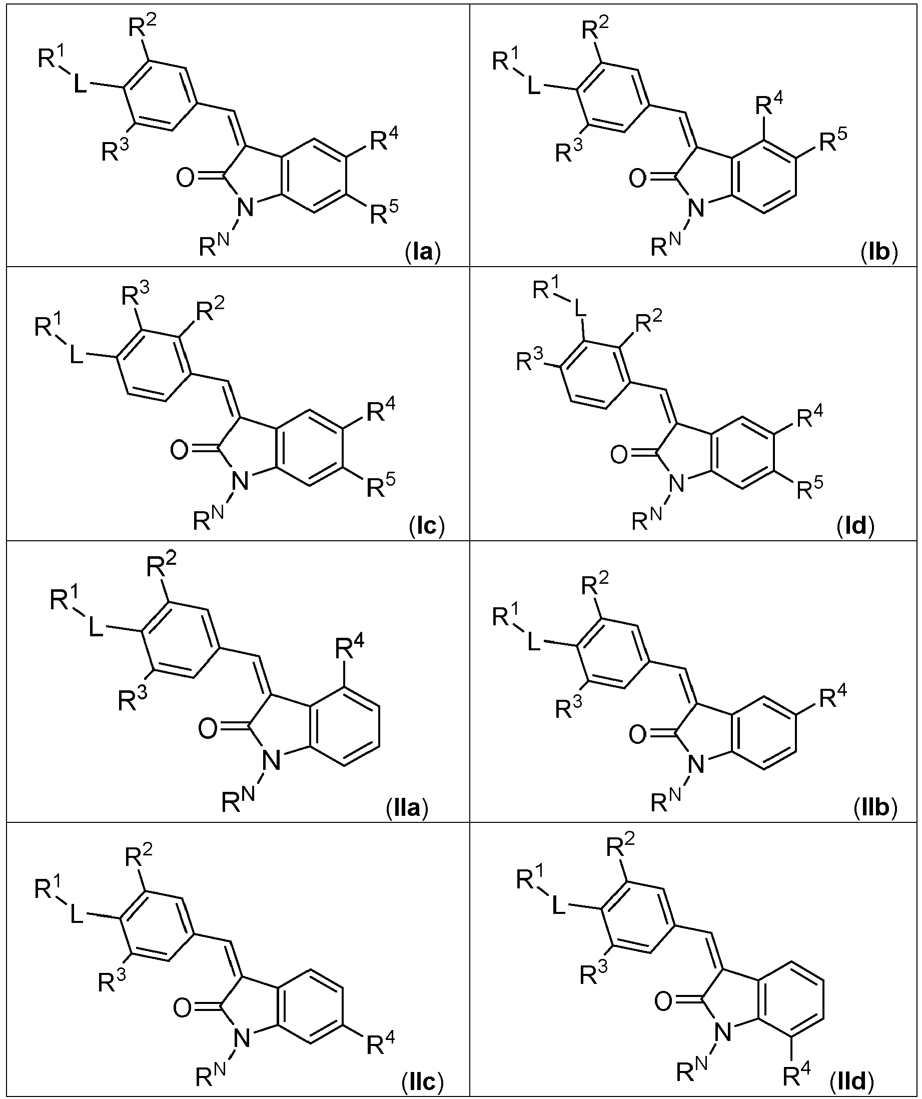

wherein **L, R¹**, **R², R³, R⁴**, **R⁵** and **R^{N}** have the same meanings as defined in the formula (I).

Preferred are also compounds of the general formula (I), wherein
**L** represents **-L₃-L₂-L₁-**;
**L₁** represents a covalent bond or -NH-CO-CH₂-O-;
**L₂** represents -(C₂H₄O)ₙ- or -(CH₂)ₘ-(OC₂H₄)ₙ-,
**L₃** represents -CO-NH- or -CH₂-CO-NH-;
**n** is an integer selected from 1, 2, 3, 4, and 5;
**m** is an integer selected from 1 or 2;
**R²** and **R³** represent independently of each other -F, -Cl, or -Br;
**R⁴** represents -F, -Cl, -Br, or -I; and
**R^{N}** represents -H, -CH₃, or -Ph.

Especially preferred are compounds of any one of the formulae (**I**), (**Ia**) - (**Id**) and (**IIaB)** - (**IId**), wherein **R¹** represents 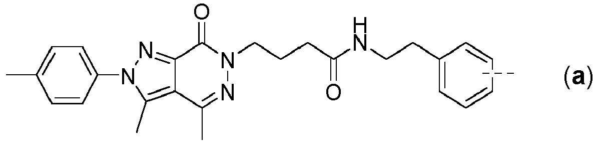 or 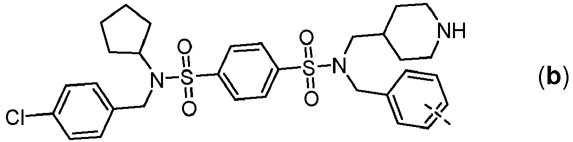 or

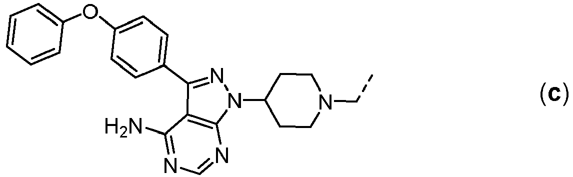

Also especially preferred are the compounds listed in the following **Table 1:**
Compound **5:** (Z)-N-(2-(2-(2-(2-(2,6-dibromo-4-((5-bromo-2-oxoindolin-3-ylidene)methyl)phenoxy) ethoxy)ethoxy)ethoxy)ethyl)-4-(2-(4-(3,4-dimethyl-7-oxo-2-(p-tolyl)-2,7-dihydro-6H-pyrazolo[3,4-d] pyridazin-6-yl)butanamido)ethyl)benzamide (**5**) 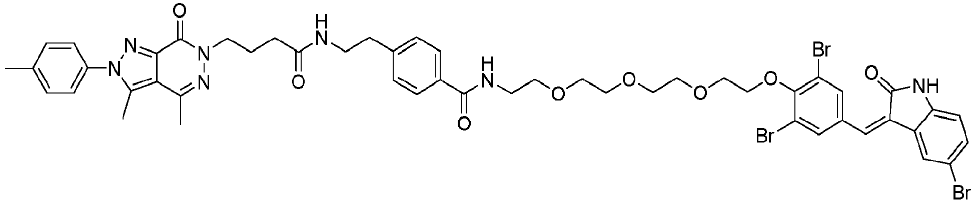
Compound **6:** (Z)-N-(2-(2-(2-(2-(2,6-dibromo-4-((5-iodo-2-oxoindolin-3-ylidene)methyl)phenoxy) ethoxy)ethoxy)ethoxy)ethyl)-4-(2-(4-(3,4-dimethyl-7-oxo-2-(p-tolyl)-2,7-dihydro-6H-pyrazolo[3,4-d]pyridazin-6-yl)butanamido)ethyl)benzamide (**6**) 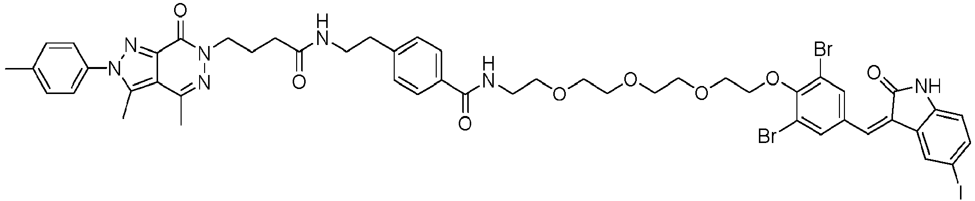
Compound **7:** (Z)-4-(((4-(N-(4-chlorobenzyl)-N-cyclopentylsulfamoyl)-N-(piperidin-4-ylmethyl) phenyl)sulfonamido)methyl)-N-(2-(2-(2-(2-(2,6-dibromo-4-((5-bromo-2-oxoindolin-3-ylidene)methyl)phenoxy)ethoxy)ethoxy)ethoxy)ethyl)benzamide (**7**) 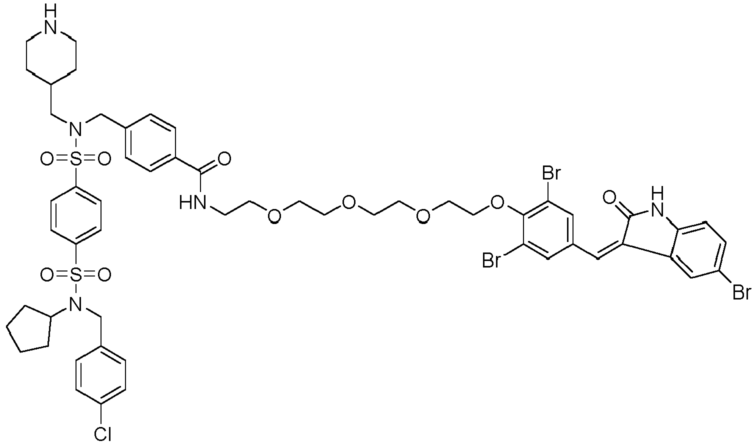
Compound **13:** (*Z*)-2-(4-(4-amino-3-(4-phenoxyphenyl)-1H-pyrazolo[3,4-d]pyrimidin-1-yl)piperidin-1-yl)-N-(1-(2,6-dibromo-4-((5-bromo-2-oxoindolin-3-ylidene)methyl)phenoxy)-2-oxo-6,9,12,15-tetr aoxa-3-azaheptadecan-17-yl)acetamide (**13**) 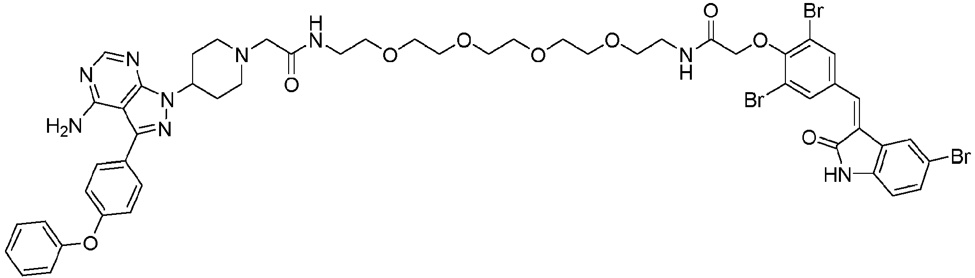
or an enantiomer, a stereoisomeric form, a mixture of enantiomers, a diastereomer, a mixture of diastereomers, a hydrate, a solvate, a racemate of the above mentioned compounds, or a pharmaceutically acceptable salts thereof.

Another aspect of the present invention is directed to a compound of the formula (I*) as an intermediate for the synthesis of a compound of the formula (I) 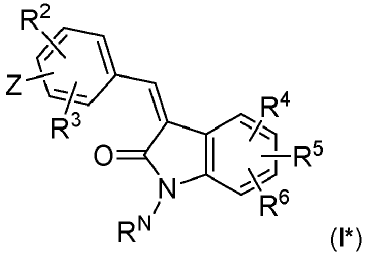 wherein
**Z** represents -OH, -O-C₂H₄-OH, -O-CH₂-COO**R'**, or **X-L₂-L₁-**;
**L₁** represents a covalent bond, -O-, -NH-, or -NH-CO-CH₂-O-;
**L₂** represents -(C₂H₄)ₙ-, -CH₂-(C₂H₄)ₙ-, -(C₂H₄O)ₙ-, -(CH₂)ₘ-(C₂H₄O)ₙ-, -(C₂H₄O)ₙ-(CH₂)ₘ-, -(CH₂)ₘ-(OC₂H₄)ₙ-, -(OC₂H₄)ₙ-(CH₂)ₘ-, -(CH₂)ₘ-(C₂H₄O)ₙ-(CH₂)ₒ-, -(CH₂)ₘ-(OC₂H₄)ₙ-(CH₂)ₒ- or -(CH₂)ₘ-(OC₂H₄)ₙ-O-(CH₂)ₒ-;
**X** represents -OH, -NH₂, -NHCO₂-C(CH₃)₃, -CO₂**R'**, -Br, -Cl, -I, or -OSO₂CH₃;
**R'** represents -H, -CH₃, -C₂H₅, -CH₂CH(CH₃)₂, or -C(CH₃)₃,
**n** is an integer selected from 1, 2, 3, 4, 5, 6, 7, 8, 9, and 10;
**m** and **o** are independently of each other an integer selected from 1, 2, 3, and 4;
**R²** and **R³** represent -Br;
**R⁴**, **R⁵**, and **R⁶** represent independently of each other -H, -F, -Br, -Cl, -I, -OH, -CN, -NO₂, -CH₃, -C₂H₅, -C₃H₇, -CH(CH₃)₂, -C₄H₉, -CH₂-CH(CH₃)₂, -cyclo-C₃H₅, -cyclo-C₄H₇, -cyclo-C₅H₉, -cyclo-C₆H₁₁, -OCH₃, -OC₂H₅, -OCOCH₃, -OC₃H₇, -O-cyclo-C₃H₅, -OCH(CH₃)₂, -OC(CH₃)₃, -OC₄H₉, -OPh, -OCH₂-Ph, -OCH₂F, -OCHF₂, -OCF₃, -CH₂F, -CHF₂, -CF₃, -CH₂-CH₂F, -CH₂-CHF₂, -CH₂-CF₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃₇ -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -S0₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, , -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, - O-CO-OC₂H₅, -O-CO-OC₃H₇, -O-CO-O-cyclo-C₃H₅, -O-CO-OCH(CH₃)₂, -O-CO-OC(CH₃)₃, -CH=CH₂, -CH₂-CH=CH₂, -C(CH₃)=CH₂, -CH=CH-CH₃, -CH=CH-Ph, -C≡CH, -C≡C-CH₃, -CH₂-C≡CH, 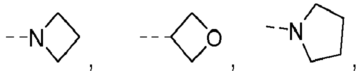 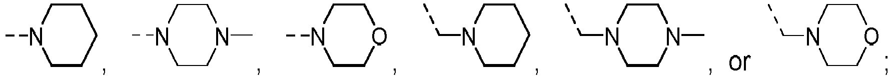
with a proviso that two of **R⁴**, **R⁵**, and **R⁶** are not -Cl at the same time;
**R^{N}** represents -H, -CH₃, -C₂H₅, -C₃H₇, -CH(CH₃)₂, -C₄H₉, -CH₂-CH(CH₃)₂, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂Ph, -COCH₃, -COCF₃, -COC₂H₅, -COC₃H₇, -COCH(CH₃)₂, -COC₄H₉, -COCH₂-CH(CH₃)₂, -COC(CH₃)₃, -CO-cyclo-C₃H₅, -CO-cyclo-C₄H₇, -CO-cyclo-C₅H₉, -CO-cyclo-C₆H₁₁, -COPh, -COCH₂Ph, -CO₂CH₃, -CO₂CF₃, -CO₂C₂H₅, -CO₂C₃H₇, -CO₂CH(CH₃)₂, -CO₂C₄H₉, -CO₂CH₂-CH(CH₃)₂, -CO₂C(CH₃)₃, -CO₂-cyclo-C₃H₅, -CO₂-cyclo-C₄H₇, -CO₂-cyclo-C₅H₉, -CO₂-cyclo-C₆H₁₁, -CO₂Ph, -CO₂CH₂Ph, -SO₂CH₃, -SO₂CF₃, -SO₂C₂H₅, -SO₂C₃H₇, -SO₂CH(CH₃)₂, -SO₂C₄H₉, -SO₂CH₂-CH(CH₃)₂, -SO₂C(CH₃)₃, -SO₂-cyclo-C₃H₅, -SO₂-cyclo-C₄H₇, -SO₂-cyclo-C₅H₉, -SO₂-cyclo-C₆H₁₁, -SO₂Ph, or -SO₂CH₂Ph;
or an enantiomer, a stereoisomeric form, a mixture of enantiomers, a diastereomer, a mixture of diastereomers, a hydrate, a solvate, a racemate of the above-mentioned compounds, or a salt thereof.

Especially preferred are the following intermediates selected from the group consisting of the compounds **2, 39** - **46, 57, 58, 61,** and **62:**

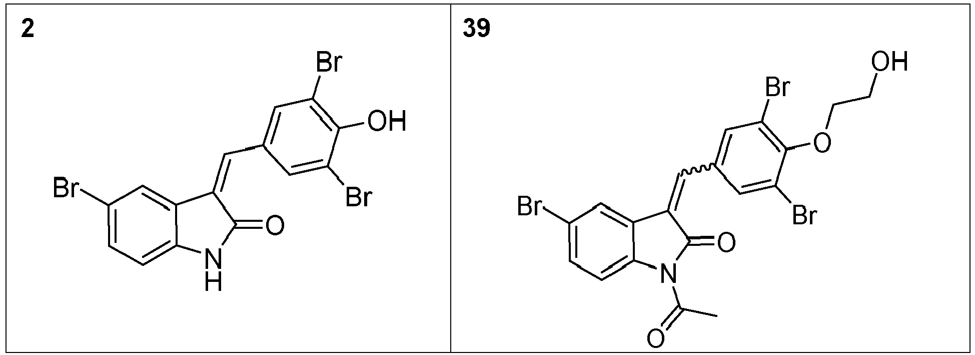

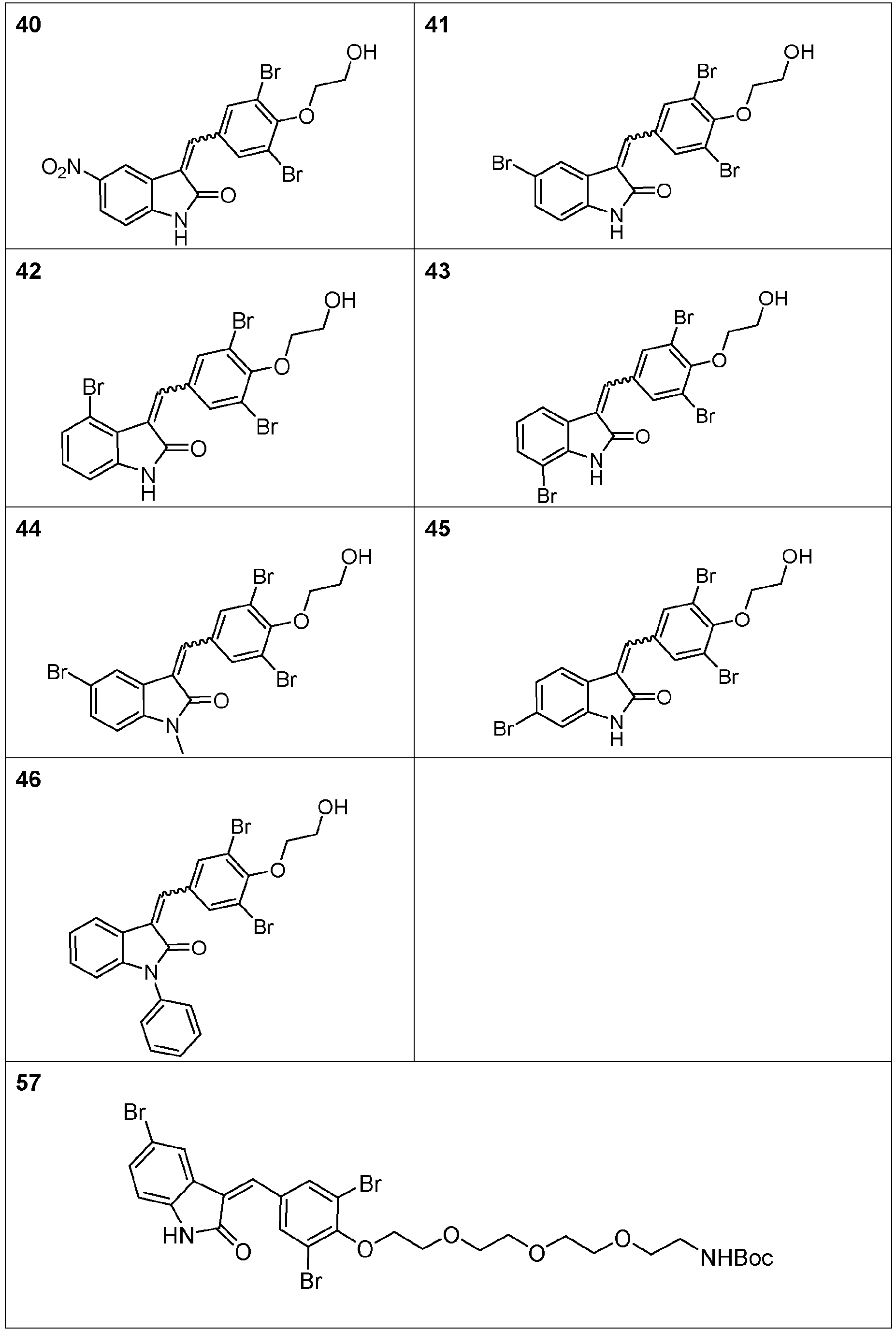

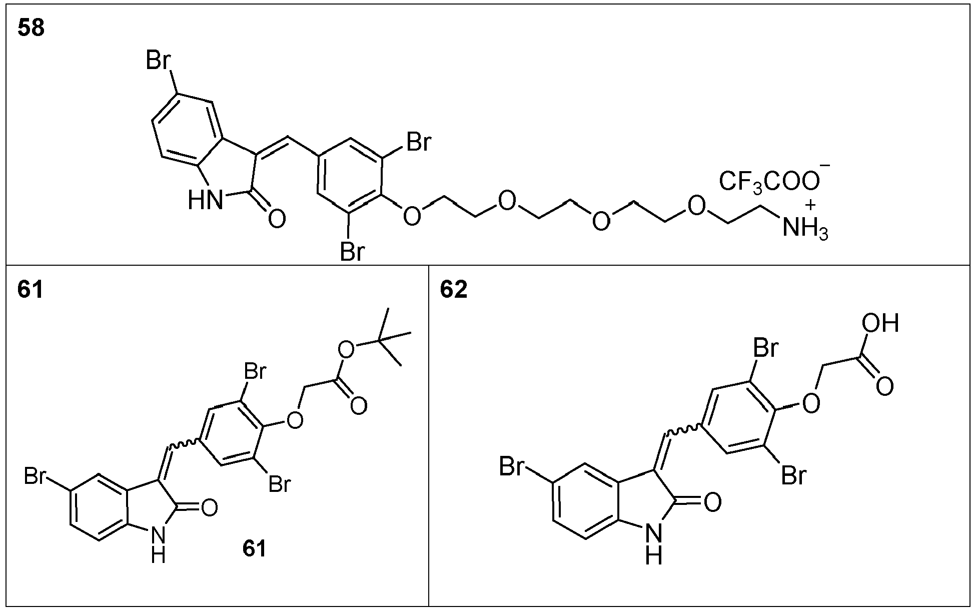

### Indications

In a further aspect of the present invention, the novel compounds according to the general formula (I) are used as pharmaceutically active agent.

Surprisingly it was found that the above-mentioned compounds of general formula (I) comprising arylidene-indolinone scaffold as DCAF11 Ligand, as well as the pharmaceutical compositions thereof are acting as bifunctional protein targeting chimeras (PROTACs), especially for degradation of PDEdelta (PDEδ) or B lymphoid *kinase* (BLK)/Bruton's tyrosine kinase (BTK).

The pharmaceutical compositions according to the present invention comprise at least one compound according to the present invention as an active ingredient together with at least one pharmaceutically acceptable (i.e. non-toxic) carrier, excipient and/or diluent.

Ras proteins, H-Ras, K-Ras and N-Ras are key regulators of diverse cellular processes including proliferation and differentiation. Ras proteins are normally tightly regulated by guanine nucleotide exchange factors (GEFs) promoting GDP dissociation and GTP binding and GTPase-activating proteins (GAPs) that stimulate the intrinsic GPTas activity of Ras to switch off signaling. Aberrant Ras function is associated with proliferative disorders, cancers and tumours. Mutation at these conserved sites favours GTP binding and produces constitutive activation of Ras.

Ras proteins are mutated in ca. 20 - 30 % of human cancers, with the K-Ras isoform most frequently mutated. However, despite substantial efforts to interfere with signaling by oncogenic Ras proteins in particular by means of Ras-farnesyltransferase inhibitors, has not led to clinically useful drugs yet. Signaling by Ras proteins critically depends on their correct subcellular localization which in turn is regulated by lipid modifications at their C-terminus. Thus, K-Ras, a major proto-oncogenic isoform of the Ras proteins, is S-farnesylated at its C-terminal CaaX box.

Oncogenic mutations in the K-Ras gene occur early during the colonic transformation process in 40% of colorectal carcinomas (CRC). One of the current clinical treatments for CRC is the administration of antibodies (cetuximab and panitumumab) that inactivate the receptor tyrosine kinase EGFR, to which 30% of the patients respond. However, CRC cells harboring oncogenic K-Ras mutation are resistant to EGFR antibody treatment and these CRC patients do not respond to the treatment.

PDEδ was originally identified as a regulatory (noncatalytic) subunit of the enzyme PDE6. PDEδ is referred to in the scientific literature using several different designations, including PDE delta, PDEG, PDE6δ, PDE6 delta, PDE66, PDE6D (Phosphodiesterase 6 delta) and PDED.

PDEδ binds K-Ras via the farnesylated C-terminus and has an essential role in sustaining its spatial organization and proper localization in cells by facilitating its intracellular diffusion to enhance the kinetics of trapping at the right membrane compartment, i.e. the plasma membrane for K-Ras. This regulation of Ras localization and signaling by PDEδ suggests that interfering with the PDEδ-Ras interaction by means of small molecules provides a novel opportunity to suppress signaling from oncogenic and normal Ras. Suppressing oncogenic Ras signaling results in halting Ras-dependent tumor proliferation.

Bruton's tyrosine kinase (BTK) is a soluble tyrosine kinase with central roles in the development, maturation, and signaling of B cells. BTK has been found to regulate cell proliferation, survival, and migration in various B-cell malignancies. Targeting BTK with recently developed BTK inhibitors has recently been approved for the treatment of several hematological malignancies and has transformed the treatment of several B-cell malignancies. The roles that BTK plays in B cells have been appreciated for some time. Recent studies have established that BTK is expressed and plays pro-tumorigenic roles in several epithelial cancers.

BTK is critical to intracellular signaling in B cells and cells of the myeloid lineage including monocytes and macrophages. BTK is a key enzyme in the B-cell receptor (BCR) signal transduction pathway and an essential signaling molecule downstream of BCR that is central to regulation of B-cell development, proliferation, activation, and differentiation into memory B cells and antibody producing plasma cells.

As BTK is involved in B-cell and other cell signaling pathways, BTKi also appear to be promising drugs for treatment of autoimmune diseases with aberrant B-cell function such as SLE, RA, and MS. BTK modulates cell signaling pathways of B cells and other myeloid cells including monocytes, macrophages and microglia that are important in the pathogenesis of MS. BTK inhibition can potentially downregulate inflammation and promote remyelination through suppression of microglial activation in the central nervous system (CNS). The importance of B cells as mediators of inflammation in MS has been well established and supported by recent successful trials of B-cell depleting agents.

Thus, in a further aspect, the present invention refers to a compound of the general formula (**I**), (**Ia**), (**Ib**), (**Ic**), (**Id**), (**IIa**) (**IIb**), (**IIc**), or (**IId**) for use as a medicament.

Further aspects of the present invention relate to the compound of the general formula (**I**), (**Ia**), (**Ib**), (**Ic**), (**Id**), (**IIa**) (**IIb**), (**IIc**), or (**IId**), or the above-mentioned pharmaceutical composition, for use in prophylaxis and/or treatment of a cancer, a tumor, an autoimmune disease, or an inflammatory disease.

Preferably, the compound of the general formula (**I**), (**Ia**), (**Ib**), (**Ic**), (**Id**), (**IIaB)** (**IIb**), (**IIc**), or (**IId**), or the above-mentioned pharmaceutical composition is useful for prophylaxis and/or treatment of a cancer, a tumor, an autoimmune disease, or an inflammatory disease, wherein the cancer, or the tumor is associated with and/or caused by PDEdelta (PDEδ) / K-Ras kinase, or B lymphoid *kinase* (BLK) /Bruton's tyrosine kinase (BTK); and/or the autoimmune disease is associated with and/or caused by B lymphoid *kinase* (BLK) / Bruton's tyrosine kinase (BTK).

In a further aspect of the present invention, methods for preventing and/or treating diseases caused by or associated with PDEdelta (PDEδ) / K-Ras kinase, or B lymphoid *kinase* (BLK) /Bruton's tyrosine kinase (BTK) in a mammal, especially in a human, are provided, which methods comprise administering to the mammal an amount of at least one compound according to the general formula (**I**), (**Ia**), (**Ib**), (**Ic**), (**Id**), (**IIaB)** (**IIb**), (**IIc**), or (**IId**) and/or pharmaceutically acceptable salts thereof, effective to prevent and/or treat the diseases caused by or associated with PDEdelta (PDEδ) / K-Ras kinase, or B lymphoid *kinase* (BLK) /Bruton's tyrosine kinase (BTK), in particular, the cancer, or the tumor is associated with and/or caused by PDEdelta (PDEδ) / K-Ras kinase, or B lymphoid *kinase* (BLK) /Bruton's tyrosine kinase (BTK); and/or
the autoimmune disease is associated with and/or caused by B lymphoid *kinase* (BLK) / Bruton's tyrosine kinase (BTK).

The term "effective amount" means an amount of compound that, when administered to a patient in need of such treatment, is sufficient to
(i) treat or prevent a particular disease, condition, or disorder which can be treated with a proteasome inhibitor;
(ii) attenuate, ameliorate, or eliminate one or more symptoms of the particular disease, condition, or disorder; or
(iii) prevent or delay the onset of one or more symptoms of the particular disease, condition, or disorder described herein.

The amount of a compound of general formula (**I**), (**Ia**), (**Ib**), (**Ic**), (**Id**), (**IIa**) (**IIb**), (**IIc**), or (**IId**) that will correspond to such an amount will vary depending upon factors such as the particular compound, disease condition and its severity, the identity (e.g., weight) of the patient in need of treatment, but can nevertheless be routinely determined by one skilled in the art.

### Cancer or Tumor

The compounds of the present application or the pharmaceutical compositions thereof are useful for the treatment and/or prophylaxis of cancer, wherein the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma, tongue cancer, astrocytomas, bronchial cancer, laryngeal cancer, malignant melanoma, oesophageal cancer, cholangiocarcinoma, and renal cell cancer.

Preferrably, the present application or the pharmaceutical composition thereof are useful for the treatment and/or prophylaxis of cancer or tumor, wherein the cancer or the tumor is selected from the group comprising or consisting of:
colorectal cancer, pancreatic cancer, lung cancer, non-small lung cancer,
prostate adenocarcinoma (PRAD), bladder adenocarcinoma (BLCA), and lung squamous (LUSC) tumors, non-Hodgkin's lymphoma (NHL), *Mantle cell lymphoma* (MCL), chronic lymphocytic leukemia (CLL), lymphoplasmacytic lymphoma, Waldenström macroglobulinemia, small lymphocytic lymphoma, cutaneous T-cell lymphoma (CTCL), acute lymphoblastic leukemia, and marginal zone lymphoma.

### Inflammatory disease

Inflammatory disease is caused by cytokines, TNF-α, IL-1β, GM-CSF, IL-6/IL-8, adhesion molecules (ICAM-1, VCAM-1, P-selectin) and prostaglandins and/or nitric oxide (NO).

### Autoimmune disease

Furthermore, the compounds of the present application or the pharmaceutical compositions thereof are useful for the treatment and/or prophylaxis of autoimmune disease. The autoimmune disease is selected from the group consisting of: Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease (AIED), Autoimmune myocarditis, Autoimmune oophoritis, Autoimmune orchitis, Autoimmune pancreatitis, Autoimmune retinopathy, Autoimmune urticaria, Axonal & neuronal neuropathy (AMAN), Baló disease, Behcet's disease, Benign mucosal pemphigoid, Bullous pemphigoid, Castleman disease (CD), Celiac disease, Chagas disease, Chronic inflammatory demyelinating polyneuropathy (CIDP), Chronic recurrent multifocal osteomyelitis (CRMO), Churg-Strauss Syndrome (CSS), Eosinophilic Granulomatosis (EGPA), Cicatricial pemphigoid, Cogan's syndrome, Cold agglutinin disease, Congenital heart block, Coxsackie myocarditis, CREST syndrome, Crohn's disease, Dermatitis herpetiformis, Dermatomyositis, Devic's disease (neuromyelitis optica), Discoid lupus, Dressler's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis, Graves' disease, Guillain-Barre syndrome, Hashimoto's thyroiditis, Hemolytic anemia, Henoch-Schonlein purpura (HSP), Herpes gestationis or pemphigoid gestationis (PG), Hidradenitis Suppurativa (HS) (Acne Inversa), Hypogammalglobulinemia, IgA Nephropathy, IgG4-related sclerosing disease, Immune thrombocytopenic purpura (ITP), Inclusion body myositis (IBM), Interstitial cystitis (IC), Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lyme disease chronic, Meniere's disease, Microscopic polyangiitis (MPA), Mixed connective tissue disease (MCTD), Mooren's ulcer, Mucha-Habermann disease, Multifocal Motor Neuropathy (MMN) or MMNCB, Multiple sclerosis, Myasthenia gravis, Myelin Oligodendrocyte Glycoprotein Antibody Disorder, Myositis, Narcolepsy, Neonatal Lupus, Neuromyelitis optica, Neutropenia, Ocular cicatricial pemphigoid, Optic neuritis, Palindromic rheumatism (PR), PANDAS, Paraneoplastic cerebellar degeneration (PCD), Paroxysmal nocturnal hemoglobinuria (PNH), Parry Romberg syndrome, Pars planitis (peripheral uveitis), Parsonage-Turner syndrome, Pemphigus, Peripheral neuropathy, Perivenous encephalomyelitis, Pernicious anemia (PA), POEMS syndrome, Polyarteritis nodosa, Polyglandular syndromes type I, II, III, Polymyalgia rheumatic, Polymyositis, Postmyocardial infarction syndrome, Postpericardiotomy syndrome, Primary Biliary Cholangitis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Sarcoidosis, Schmidt syndrome, Scleritis, Scleroderma, Sjögren's syndrome, Sperm & testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Thyroid eye disease (TED), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo, Vogt-Koyanagi-Harada Disease.

Preferred, the autoimmune disease is selected from the group comprising or consisting of: *rheumatoid arthritis (RA),* multiple sclerosis (MS), systemic lupus erythematosus (SLE), Sjogren's syndrome (SS), other B cell-associated autoimmune disorders; autoimmune haematologic conditions, primarily immune thrombocytopenia (ITP):
The compounds enlisted explicitly in **Table 1** are preferred to be used within the methods or indications disclosed herein.

### Pharmaceutical compositions

The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the compounds of the formula (**I**), (**Ia**), (**Ib**), (**Ic**), (**Id**), (**IIaB)** (**IIb**), (**IIc**), or (**IId**) or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to ca. 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### Chemical synthesis

### General information

Unless otherwise noted, all commercially available compounds and (anhydrous) solvents were purchased from Sigma Aldrich, TCI Europe, Alfa Aesar, or Acros Organic, and used without further purification. Analytical thin-layer chromatography (TLC) was performed on Merck silica gel aluminum plates with F-254 indicator. Compounds were visualized by irradiation with UV light or potassium permanganate staining followed by heating with a heat gun. Column chromatography was performed using silica gel Merck 60 (particle size 0.040-0.063 mm). Solvent mixtures are understood as volume/volume. Preparative HPLC-MS: Separations were carried out using a preparative mass-directed HPLC (Agilent Series, 1100/LC/MSD VL, Agilent Series) with a reversed-phase C18 column (flow 20.0 mL/min, solvent A: 0.1% TFA in water, solvent B: 0.1% TFA in acetonitrile).

¹H NMR, ¹³C NMR spectra were recorded at room temperature on a Bruker DRX400 (400 MHz), INOVA500 (500 MHz), INOVA 600 or INOVA 700 and referenced to the residual deuterated solvent signals. All reported NMR values are given in parts per million (ppm). Multiplicities are indicated s (singlet), d (doublet), t (triplet), q (quartet), m (multiplet); coupling constants (*J*) are given in Hertz (Hz).

High resolution mass spectra were recorded on a *LTQ Orbitrap* mass spectrometer coupled to an Accela HPLC-System (HPLC column: Hypersyl GOLD, 50 mm x 1 mm, particle size 1.9 µm, ionization method: electron spray ionization).

**Abbreviations used in the description of the chemistry and in the Examples that follow are:** MeCN (acetonitrile); br (broad); BOC (tet-butyloxycarbonyl), Cbz (benzyloxycarbonyl), CDCl₃ (deuterated chloroform); DCM (dichloromethane); DIEA (N,N-Diisopropylethylamine), DIPEA (di-iso-propylethylamine); DMF (dimethylformamide); DMSO (dimethyl sulfoxide); eq. (equivalent); EtOAc (ethyl acetate); EtOH (ethanol); HATU (*O*-(7-azabenzotriazol-1-yl)-*N*,*N*,*N*',*N*'-tetramethyluronium hexafluorophosphate); HCl (hydrochloric acid); HOBt (Hydroxybenzotriazole), MeOH (methanol); MS (mass spectrometry); Mwt (molecular weight); NMR (nuclear magnetic resonance); PE (petroleum ether); RT/r.t. (room temperature); sat. (saturated), aq. (aqueous); TFA (trifluoroacetic acid).

### Example A-1: Preparation of (Z)-N-(2-(2-(2-(2-(2,6-dibromo-4-((5-bromo-2-oxoindolin-3-ylidene)methyl)phenoxy) ethoxy)ethoxy)ethoxy)ethyl)-4-(2-(4-(3,4-dimethyl-7-oxo-2-(p-tolyl)-2,7-dihydro-6H-pyrazolo[3,4-d] pyridazin-6-yl)butanamido)ethyl)benzamide (5)

### 5-bromo-3-(3,5-dibromo-4-hydroxybenzylidene)indolin-2-one (2)

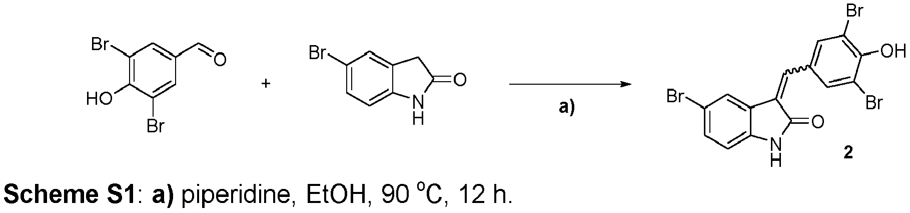

**a):** Piperidine (5 µL, 0.05 mmol, 0.1 eq.) was added into a solution of 5-bromooxindole (0.106 g, 0.5 mmol, 1 eq.) and 3,5-dibromo-4-hydroxybenzaldehyde (0.154 g, 0.55 mmol, 1 eq.) in EtOH (6 mL) and the solution was stirred for 12 h at 90 °C. The solution was cooled to room temperature, filtered, and the filter cake was washed with cold MeOH (5 mL) for two times to provide **2** as a yellow solid (0.109 g, 46%).

**¹H NMR** (500 MHz, DMSO-*d*₆) δ 10.78 - 10.70 (s, 1H), 8.79 (s, 1.28H), 7.93 (s, 0.66H), 7.87 (d, *J* = 2.0 Hz, 0.64H), 7.79 (s, 0.64H), 7.62 (d, *J* = 1.9 Hz, 0.36H), 7.55 (s, 0.36H), 7.42 (dd, *J* = 8.4, 2.0 Hz, 0.36H), 7.34 (dd, *J* = 8.2, 2.0 Hz, 0.64H), 6.85 (d, *J* = 8.3 Hz, 0.36H), 6.78 (d, *J* = 8.2 Hz, 0.64H).

**¹³C NMR** (126 MHz, DMSO-*d*₆) δ 168.07, 166.97, 142.01, 139.33, 136.56, 136.24, 135.16, 133.45, 132.24, 130.54, 127.52, 124.41, 123.02, 122.10, 113.02, 112.58, 112.04, 111.58, 111.19.

**HRMS** calculated for C₁₅H₉Br₂⁸¹BrNO₂ [M + H]⁺: 473.8158, found 473.8157.

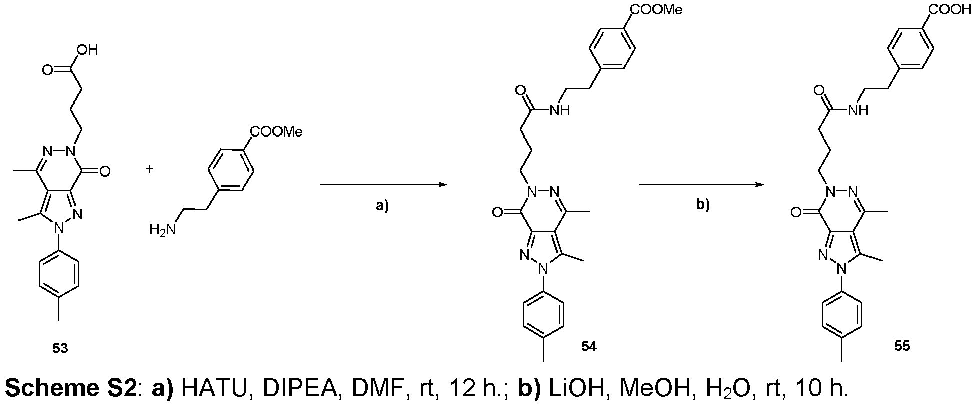

Compound **53** was synthesized according to the literature².
**a):** DIEAP (1.046 mL, 6 mmol, 3 eq.) was added into the mixture of compound **53** (0.6808 g, 2 mmol, 1 eq.) and HATU (0.912 g, 2.4 mmol, 1.2 eq.) in DMF (6 mL) and stirred for 10 min. After that, methyl 4-(2-amino-ethyl)-benzoate (0.43 g, 2.4 mmol, 1.2 eq.) was added into the resulting solution and stirred at room temperature for 4 h. The reaction mixture was diluted with EtOAc, washed with water and brine, dried over Na₂SO₄. After removing the solvent under reduced pressure, the product **54** (0.5919 g, 59%) could be obtained by flash chromatography (DCM : MeOH = 40 : 1) as a light yellow solid.

### methyl 4-(2-(4-(3.4-dimethyl-7-oxo-2-(p-tolyl)-2.7-dihydro-6H-pyrazolo[3.4-d]pyrida zin-6-yl)butanamido)ethyl)benzoate (54)

**¹H NMR** (700 MHz, CDCl₃) δ 7.93 (d, *J* = 8.3 Hz, 2H), 7.37 - 7.32 (m, 4H), 7.30 (d, *J* = 8.2 Hz, 2H), 4.13 (t, *J* = 6.0 Hz, 2H), 3.88 (s, 3H), 3.55 (q, *J* = 7.0 Hz, 2H), 2.96 (t, *J* = 7.5 Hz, 2H), 2.63 (s, 3H), 2.55 (s, 3H), 2.45 (s, 3H), 2.19 (dd, *J* = 7.9, 5.2 Hz, 2H), 2.12 (q, *J* = 5.9 Hz, 2H).

**¹³C NMR** (176 MHz, CDCl₃) δ 172.97, 167.14, 157.14, 144.88, 141.98, 141.84, 140.03, 136.57, 135.95, 130.00, 129.81, 128.95, 128.26, 125.83, 117.84, 52.04, 48.61, 40.50, 35.77, 33.46, 25.70, 21.33, 19.97, 12.39.

**MS** calculated for C₂₈H₃₂N₅O₄ [M + H]⁺: 502.2, found 502.2.

**b):** Compound **54** (0.5016 g, 1 mmol, 1 eq.) was added to the solution of LiOH (72 mg, 3 mmol, 3 eq.) in MeOH/H₂O (v/v = 5/1, 4 mL). After stirring for 10 h, the reaction mixture was diluted with EtOAc, and acidified with 1 M HCl. The aqueous layer was extracted with EtOAc, and the combined organic layer was dried over anhydrous MgSO₄ and concentrated under vacuum. The obtained product **55** (40 mg, 82%) can be used for next step without further purification.

### 4-(2-(4-(3,4-dimethyl-7-oxo-2-(p-tolyl)-2.7-dihydro-6H-pyrazolo[3,4-d]pyridazin-6-yl)butanamido)ethyl)benzoic acid (55)

**¹H NMR** (400 MHz, DMSO-*d*₆) δ 12.71 (s, 1H), 7.84 (t, *J* = 5.6 Hz, 1H), 7.80 - 7.73 (m, 2H), 7.43 - 7.33 (m, 4H), 7.24 (d, *J* = 8.3 Hz, 2H), 3.94 (t, *J* = 7.0 Hz, 2H), 3.21 (q, *J* = 7.1 Hz, 2H), 2.69 (t, *J* = 7.2 Hz, 2H), 2.51 (s, 3H), 2.44 (s, 3H), 2.35 (s, 3H), 2.01 (t, *J* = 7.6 Hz, 2H), 1.87 - 1.77 (m, 2H).

**¹³C NMR** (101 MHz, DMSO-*d*₆) δ 171.42, 167.26, 155.15, 144.91, 141.13, 140.98, 139.34, 137.41, 135.89, 129.89, 129.34, 128.87, 128.64, 125.72, 117.13, 48.52, 35.08, 32.61, 24.61, 20.74, 19.42, 11.86.

**HRMS** calculated for C₂₇H₃₀N₅O₄ [M + H]⁺: 488.2292, found 488.2287.

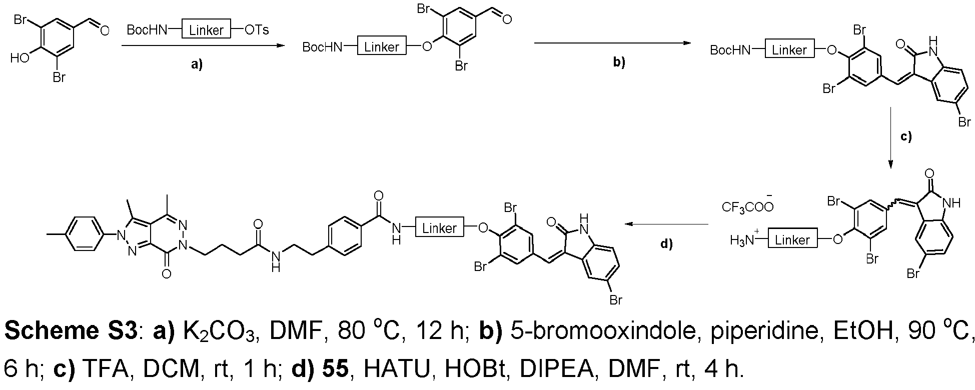

### tert-butyl (2-(2-(2-(2-(2.6-dibromo-4-formylphenoxy)ethoxy)ethoxy)ethoxy)ethyl) carbamate (56)

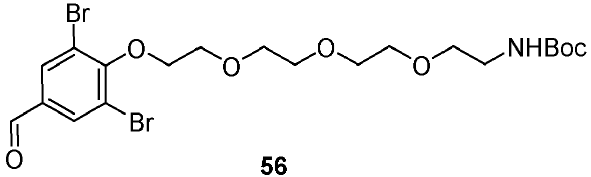

**a):** To a 25 mL flask, 3,5-dibromo-4-hydroxybenzaldehyde (0.28 g, 1 mmol, 1 eq.), *t-*Boc-*N*-amido-PEG4-Tos (0.5818 g, 1.3 mmol, 1.3 eq.), K₂CO₃ (0.4146 g, 3 mmol, 3 eq.) and anhydrous DMF (3 mL) were added. The resulting mixture was stirred at 80 °C for 12 h. After cooled to room temperature, the residue was dissolved in EtOAc, washed successively with water and brine. The organic layer was combined, dried over anhydrous Na₂SO₄, and concentrated under reduced pressure. The crude product was subjected to flash chromatography (pentane : EtOAc = 2 : 1). The final product **56** (0.3387 g, 70%) was obtained as a light yellow oil.

**¹H NMR** (400 MHz, CDCl₃) δ 9.85 (s, 1H), 8.02 (s, 2H), 4.32 - 4.25 (m, 2H), 3.98 - 3.87 (m, 2H), 3.80 - 3.74 (m, 2H), 3.70 - 3.60 (m, 6H), 3.53 (t, *J* = 5.1 Hz, 2H), 3.30 (t, *J* = 4.3 Hz, 2H), 1.43 (s, 9H).

**¹³C NMR** (101 MHz, CDCl₃) δ 188.52, 158.44, 156.13, 134.27, 134.07, 119.46, 79.39, 73.12, 71.05, 70.82, 70.77, 70.44, 70.39, 70.25, 40.70, 28.57.

**HRMS** calculated for C₂₀H₃₀Br₂NO₇ [M + H]⁺: 554.0384, found 554.0390.

### tert-butyl (Z)-(2-(2-(2-(2-(2,6-dibromo-4-((5-bromo-2-oxoindolin-3-ylidene)methyl) phenoxy)ethoxy)ethoxy)ethoxy)ethyl)carbamate (57)

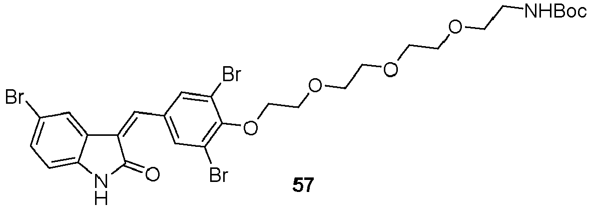

**b):** Piperidine (2 µL, 0.02 mmol, 0.1 eq.) were added into the solution of 5-bromooxindole (42.4 mg, 0.2 mmol, 1 eq.) and compound **56** (122.2 mg, 0.22 mmol, 1.1 eq.) in EtOH (3 mL) and stirred for 6 h at 90 °C, then cooled to room temperature. After removing the solvent under reduced pressure, the product **57** (76.4 mg, 51%) could be obtained by flash chromatography (pentane : EtOAc = 2 : 3) as a red solid.

**¹H NMR** (400 MHz, DMSO-*d*₆) δ 10.82 (s, 1H), 8.76 (s, 2H), 7.94 - 7.78 (m, 2H), 7.38 (dd, *J* = 8.3, 2.0 Hz, 1H), 6.78 (d, *J* = 8.2 Hz, 1H), 6.70 (d, *J* = 6.2 Hz, 1H), 4.17 (t, *J =* 4.7 Hz, 2H), 3.81 (t, *J* = 4.7 Hz, 2H), 3.64 - 3.56 (m, 2H), 3.56 - 3.44 (m, 6H), 3.35 (t, *J* = 6.1 Hz, 2H), 3.03 (q, *J* = 6.0 Hz, 2H), 1.34 (s, 9H).

**¹³C NMR** (126 MHz, DMSO-*d*₆) δ 166.68, 155.55, 154.18, 140.06, 136.20, 134.85, 132.64, 131.69, 126.91, 126.81, 122.86, 117.23, 113.21, 111.50, 77.55, 72.84, 69.93, 69.78, 69.50, 69.15, 28.22.

**HRMS** calculated for C₂₈H₃₄Br₃N₂O₇ [M + H]⁺: 746.9911, found 746.9915.

### (Z)-N-(2-(2-(2-(2-(2.6-dibromo-4-((5-bromo-2-oxoindolin-3-ylidene)methyl)phenoxy) ethoxy)ethoxy)ethoxy)ethyl)-4-(2-(4-(3,4-dimethyl-7-oxo-2-(p-tolyl)-2,7-dihydro-6H-pyrazolo[3,4-d] pyridazin-6-yl)butanamido)ethyl)benzamide (5)

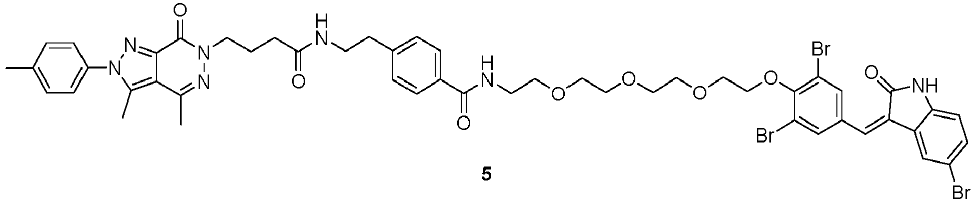

**c-d):** Compound **57** (45 mg, 0.06 mmol, 1.2 eq.) was added into a solution of TFA (1 mL) with DCM (1 mL). After stirring at room temperature for 1 h, the mixture was evaporated to remove the solvent, affording the amine intermediate **58** without further purification.

The mixture of compound **3** (24.4 mg, 0.05 mmol, 1 eq.), DIPEA (52.3 µL, 0.3 mmol, 6 eq.), HOBT (8.8 mg, 0.065 mmol, 1.3 eq.) and HATU (22.8 mg, 0.06 mmol, 1.2 eq.) in DMF (2 mL) was stirred for 10 min. After that, intermediate **58** was added into the resulting solution and stirred at room temperature for 4 h. The reaction mixture was diluted with EtOAc, washed with water and brine, dried over Na₂SO₄, and concentrated in vacuo. Purification by preparative HPLC elution with 10-100% MeCN in H₂O (with 0.1% TFA) afforded the trifluoroacetic acid salt of (Z)-stereoisomer **5** (12.9 mg, 21%) as a yellow solid, which was then used for biological evaluation. Additionally, isomerization was detected when compound **5** was incubated in phosphate-buffered saline (PBS)/MeCN (v/v = 1/1) as a 100 µM solution at 37 °C, in which *Z*/*E* ratio decreased to 4.7 : 1 after 6 h and 1.2 : 1 after 18 h, respectively.

¹H **NMR** (400 MHz, DMSO-*d*₆) δ 10.81 (s, 1H), 8.74 (s, 2H), 8.38 (t, *J* = 5.6 Hz, 1H), 7.90 - 7.80 (m, 3H), 7.74 (d, *J* = 8.2 Hz, 2H), 7.47 - 7.38 (m, 4H), 7.36 (dd, *J* = 8.3, 2.0 Hz, 1H), 7.24 (d, *J* = 8.2 Hz, 2H), 6.77 (d, *J* = 8.3 Hz, 1H), 4.14 (t, *J* = 4.7 Hz, 2H), 3.99 (t, *J* = 7.0 Hz, 2H), 3.81 - 3.76 (m, 2H), 3.60 - 3.55 (m, 2H), 3.53 - 3.47 (m, 8H), 3.37 (q, *J* = 5.9 Hz, 2H), 3.25 (q, *J* = 6.9 Hz, 2H), 2.71 (t, *J* = 7.2 Hz, 2H), 2.55 (s, 3H), 2.48 (s, 3H), 2.40 (s, 3H), 2.10 - 2.03 (m, 2H), 1.88 (p, *J* = 7.6 Hz, 2H).

**¹³C NMR** (176 MHz, DMSO-*d*₆) δ 171.40, 166.66, 166.09, 155.14, 154.15, 142.88, 141.12, 140.94, 140.04, 139.31, 137.35, 137.34, 136.19, 135.87, 134.82, 132.63, 132.27, 131.65, 129.87, 128.47, 127.20, 126.89, 126.80, 125.68, 122.83, 117.20, 117.13, 113.21, 111.48, 72.81, 69.92, 69.79, 69.78, 69.62, 69.48, 68.92, 48.55, 34.93, 32.62, 24.62, 20.73, 19.40, 11.85.

**HRMS** calculated for C₅₀H₅₃Br₃N₇O₈ [M + H]⁺: 1116.1500, found 1116.1532.

### HPLC method for stability test

| Time (min) | %A | %B |
|---|---|---|
| 0 | 90 | 10 |
| 1 | 90 | 10 |
| 2 | 40 | 60 |
| 7 | 20 | 80 |
| 8 | 5 | 95 |
| 10 | 5 | 95 |
| 12 | 90 | 10 |

| | | |
|---|---|---|
| A: ddH₂O + 0.1% (v/v) formic acid; B: acetonitrile + 0.1% (v/v) formic acid; flow rate:0.25 mL/min. | | |

Instruments: Velos Pro (Thermo Fisher Scientific, US), Ultimate 3000 HPLC (Thermo Fisher Scientific, US).

### Example A-2: Preparation of (Z)-N-(2-(2-(2-(2-(2,6-dibromo-4-((5-iodo-2-oxoindolin-3-ylidene)methyl)phenoxy) ethoxy)ethoxy)ethoxy)ethyl)-4-(2-(4-(3,4-dimethyl-7-oxo-2-(p-tolyl)-2,7-dihydro-6H-pyrazolo[3,4-d]pyridazin-6-yl)butanamido)ethyl) benzamide (6)

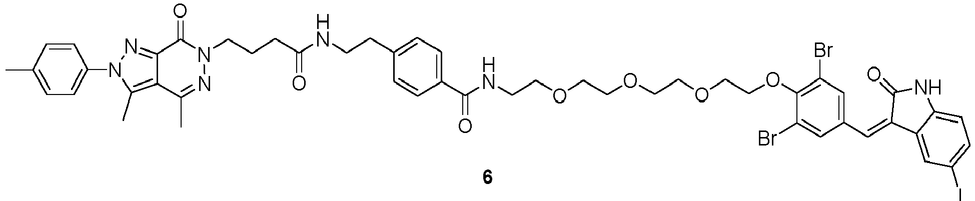

Compound **6** (7.6 mg, 13%) was obtained as a yellow solid.

**¹H NMR** (600 MHz, DMSO-*d*₆) δ 10.83 (s, 1H), 8.78 (s, 2H), 8.41 (t, *J* = 5.6 Hz, 1H), 8.04 (d, *J* = 1.7 Hz, 1H), 7.89 (t, *J* = 5.6 Hz, 1H), 7.85 (s, 1H), 7.76 (d, *J* = 8.3 Hz, 2H), 7.55 (dd, *J* = 8.1, 1.7 Hz, 1H), 7.51 - 7.46 (m, 2H), 7.43 (d, *J* = 8.3 Hz, 2H), 7.27 (d, *J =* 8.2 Hz, 2H), 6.69 (d, *J* = 8.1 Hz, 1H), 4.19 -4.14 (m, 2H), 4.02 (t, *J* = 7.1 Hz, 2H), 3.82 - 3.80 (m, 2H), 3.60 (dd, *J* = 6.0, 3.7 Hz, 2H), 3.55 - 3.51 (m, 8H), 3.39 (q, *J* = 5.9 Hz, 2H), 3.27 (q, *J* = 6.9 Hz, 2H), 2.74 (t, *J* = 7.2 Hz, 2H), 2.59 (s, 3H), 2.43 (s, 3H), 2.09 (t, *J* = 7.6 Hz, 2H), 1.93 - 1.87 (m, 2H).

**¹³C NMR** (151 MHz, DMSO-*d*₆) δ 171.38, 166.49, 166.08, 155.14, 154.11, 142.88, 141.12, 140.96, 140.48, 139.33, 137.46, 137.38, 136.18, 135.87, 134.59, 132.70, 132.26, 129.88, 128.48, 128.39, 127.20, 127.12, 126.72, 125.70, 117.20, 117.13, 111.95, 84.19, 72.81, 69.92, 69.78, 69.62, 69.48, 68.92, 48.54, 34.93, 32.61, 24.62, 20.74, 19.42, 11.86.

**HRMS** calculated for C₅₀H₅₃Br₂IN₇O₈ [M + H]⁺: 1164.1362, found 1164.1383.

### Example A-3: Preparation of (Z)-4-(((4-(N-(4-chlorobenzyl)-N-cyclopentylsulfamoyl)-N-(piperidin-4-ylmethyl) phenyl)sulfonamido)methyl)-N-(2-(2-(2-(2-(2,6-dibromo-4-((5-bromo-2-oxoindolin-3-ylidene)methyl)phenoxy)ethoxy) ethoxy)ethoxy)ethyl)benzamide (7)

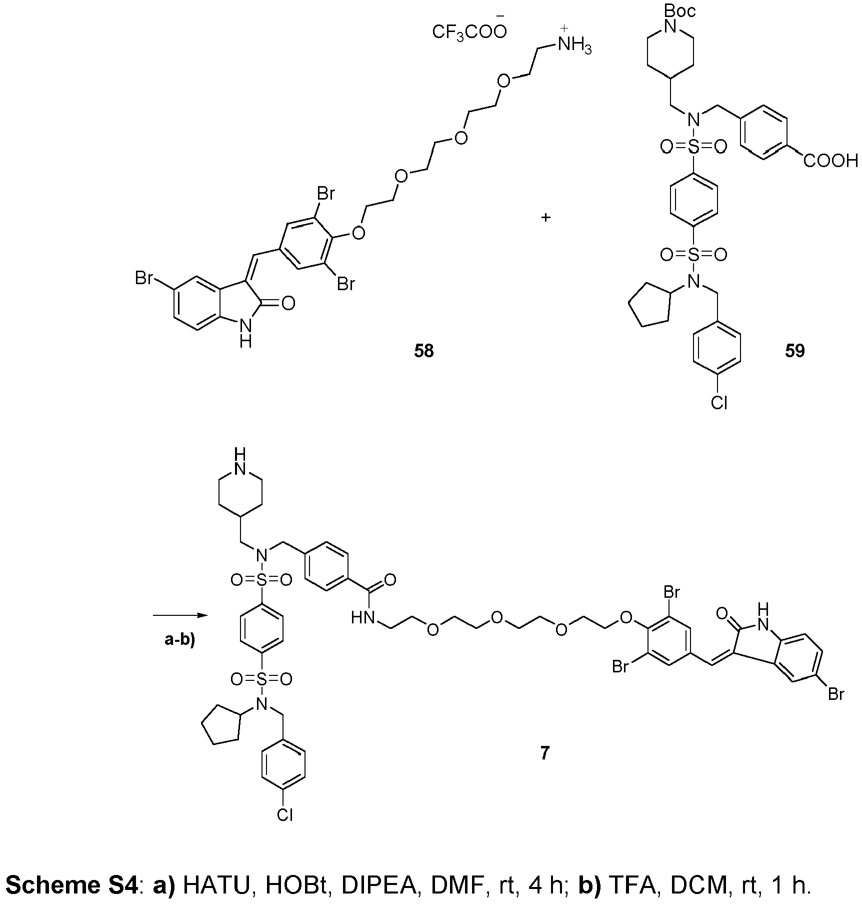

Compound **59** was synthesized according to the literature³.

Compound **57** (45 mg, 0.06 mmol, 1.2 eq.) was added into a solution of TFA (1 mL) with DCM (1 mL). After stirring at room temperature for 1 h, the mixture was evaporated to remove the solvent, affording the amine intermediate **58** without further purification.
**a):** The mixture of compound **59** (38 mg, 0.05 mmol, 1 eq.), DIPEA (52.3 µL, 0.3 mmol, 6 eq.), HOBT (8.8 mg, 0.065 mmol, 1.3 eq.) and HATU (22.8 mg, 0.06 mmol, 1.2 eq.) in DMF (2 mL) was stirred for 10 min. After that, intermediate **58** was added into the resulting solution and stirred at room temperature for 4 h. The reaction mixture was diluted with EtOAc, washed with water and brine, dried over Na₂SO₄, and concentrated in vacuo. The product could be obtained by flash chromatography (DCM : MeOH = 20 : 1) as a yellow oil.
**b):** Afterwards, the product was dissolved into a solution of TFA (1 mL) with DCM (1 mL). After stirring at room temperature for 1 h, the mixture was evaporated to remove the solvent. Trifluoroacetic acid salt of **7** (12.6 mg, 18%) was obtained as a yellow solid by preparative HPLC with mobile phase of water and MeCN (0.1 % TFA).

**¹H NMR** (700 MHz, DMSO-*d*₆) δ 10.79 (s, 1H), 8.71 (s, 2H), 8.50 (d, *J* = 11.2 Hz, 1H), 8.45 (t, *J* = 5.6 Hz, 1H), 8.18 (q, *J =* 11.0 Hz, 1H), 8.06 - 7.96 (m, 4H), 7.83 (d, *J* = 2.0 Hz, 1H), 7.80 (s, 1H), 7.74 (d, *J* = 8.2 Hz, 2H), 7.40 - 7.30 (m, 5H), 7.26 (d, *J* = 8.1 Hz, 2H), 6.74 (d, *J* = 8.2 Hz, 1H), 4.33 (d, *J* = 8.5 Hz, 4H), 4.24 - 4.18 (m, 1H), 4.12 - 4.08 (m, 2H), 3.78 - 3.72 (m, 2H), 3.55 - 3.52 (m, 2H), 3.50 - 3.42 (m, 8H), 3.34 (q, *J* = 5.9 Hz, 2H), 3.16 - 3.09 (m, 2H), 2.99 (d, *J* = 7.3 Hz, 2H), 2.58 (q, *J* = 12.0 Hz, 2H), 1.66 - 1.50 (m, 3H), 1.48 - 1.34 (m, 4H), 1.33 - 1.24 (m, 2H), 1.15 - 1.00 (m, 4H).

**¹³C NMR** (176 MHz, DMSO-*d*₆) δ 166.69, 165.78, 158.42, 158.23, 158.05, 157.86, 154.15, 143.69, 142.53, 140.07, 139.45, 138.36, 136.20, 134.81, 133.72, 132.66, 131.70, 131.53, 128.67, 128.23, 128.17, 128.14, 128.04, 127.35, 126.94, 126.81, 122.85, 117.21, 113.23, 111.52, 72.82, 69.92, 69.78, 69.61, 69.49, 68.90, 59.14, 53.38, 51.73, 46.14, 42.64, 31.73, 28.61, 25.90, 22.89.

**HRMS** calculated for C₅₅H₆₂Br₃ClN₅O₁₀S₂ [M + H]⁺: 1288.1171, found 1288.1211.

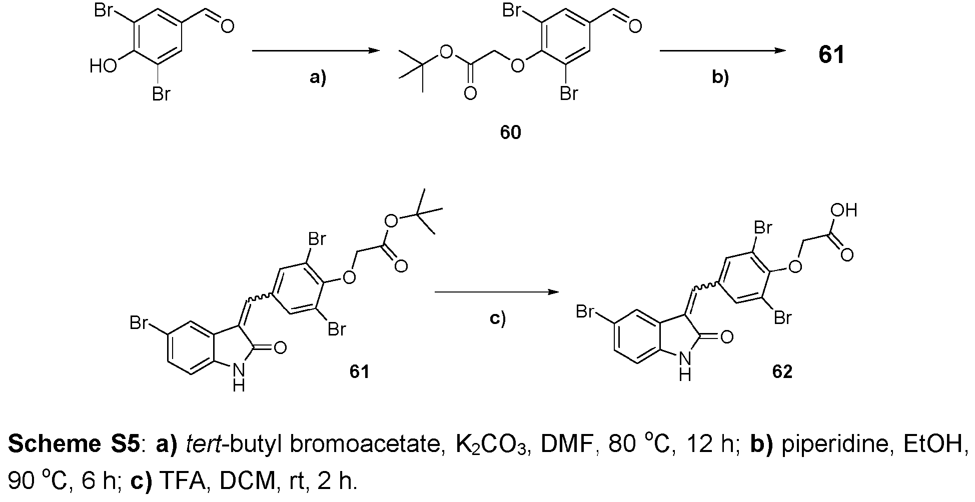

### tert-butyl 2-(2,6-dibromo-4-formylphenoxy)acetate (60)

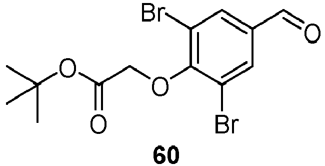

**a):** To a 25 mL flask, 3,5-dibromo-4-hydroxybenzaldehyde (0.56 g, 2 mmol, 1 eq.), *tert-*butyl bromoacetate (0.5852 g, 3 mmol, 1.5 eq.), K₂CO₃ (0.8292 g, 6 mmol, 3 eq.) and anhydrous DMF (5 mL) were added. The resulting mixture was stirred at 80 °C for 12 h.

After cooled to room temperature, the residue was dissolved in EtOAc, washed successively with water and brine. The organic layer was combined, dried over anhydrous Na₂SO₄, and concentrated under reduced pressure. The crude product was subjected to flash chromatography (pentane : EtOAc = 6 : 1). The final product **60** (0.654 g, 83%) was obtained as a white solid.

**¹H NMR** (400 MHz, CDCl₃) δ 9.86 (s, 1H), 8.03 (s, 2H), 4.60 (s, 2H), 1.53 (s, 9H).

**¹³C NMR** (126 MHz, CDCl₃) δ 188.45, 166.37, 157.39, 134.53, 134.06, 119.08, 82.85, 69.68, 28.21.

**HRMS** calculated for C₁₃H₁₅Br₂O₄ [M + H]⁺: 392.9332, found 392.9337.

### tert-butyl 2-(2.6-dibromo-4-((5-bromo-2-oxoindolin-3-ylidene)methyl)phenoxy) acetate (61)

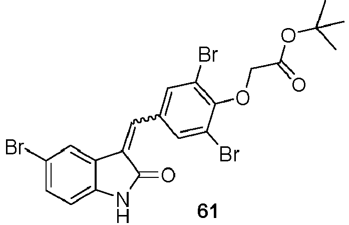

**b):** Piperidine (2 µL, 0.02 mmol, 0.1 eq.) were added into the solution of 5-bromooxindole (42.4 mg, 0.2 mmol, 1 eq.) and compound **60** (86.7 mg, 0.22 mmol, 1.1 eq.) in EtOH (3 mL) and stirred for 6 h at 90 °C, then cooled to room temperature. After removing the solvent under reduced pressure, the product **61** (89.4 mg, 76%) could be obtained by flash chromatography (pentane : EtOAc = 3 : 1) as a deep yellow solid.

**¹H NMR** (600 MHz, DMSO-*d*₆) δ 10.81 (s, 1H), 8.77 (s, 1H), 8.01 (s, 0.85H), 7.86 (d, *J* = 23.7 Hz, 1.13H), 7.55 (d, *J* = 34.3 Hz, 0.87H), 7.40 (d, 8.3 Hz, 1H), 6.81 (d, *J* = 7.7 Hz, 1H), 4.60 (s, 2H), 1.48 (s, 9H).

**¹³C NMR** (151 MHz, DMSO-*d*₆) δ 167.73, 166.62, 166.20, 166.18, 152.93, 152.48, 142.40, 140.07, 136.17, 134.64, 133.82, 133.45, 133.31, 132.99, 132.88, 131.69, 128.17, 127.10, 126.75, 124.83, 122.85, 122.54, 117.53, 116.80, 113.21, 112.67, 112.19, 111.46, 81.74, 69.49, 69.45, 27.70.

**HRMS** calculated for C₂₁H₁₉Br₃NO₄ [M + H]⁺: 585.8859, found 585.8858.

### Example A-4: Preparation of (Z)-2-(4-(4-amino-3-(4-phenoxyphenyl)-1H-pyrazolo[3,4-d]pyrimidin-1-yl)piperidin-1-yl)-N-(1-(2,6-dibromo-4-((5-bromo-2-oxoindolin-3-ylidene)methyl)phenoxy)-2-oxo-6,9,12,15-tetr aoxa-3-azaheptadecan-17-yl)acetamide (13)

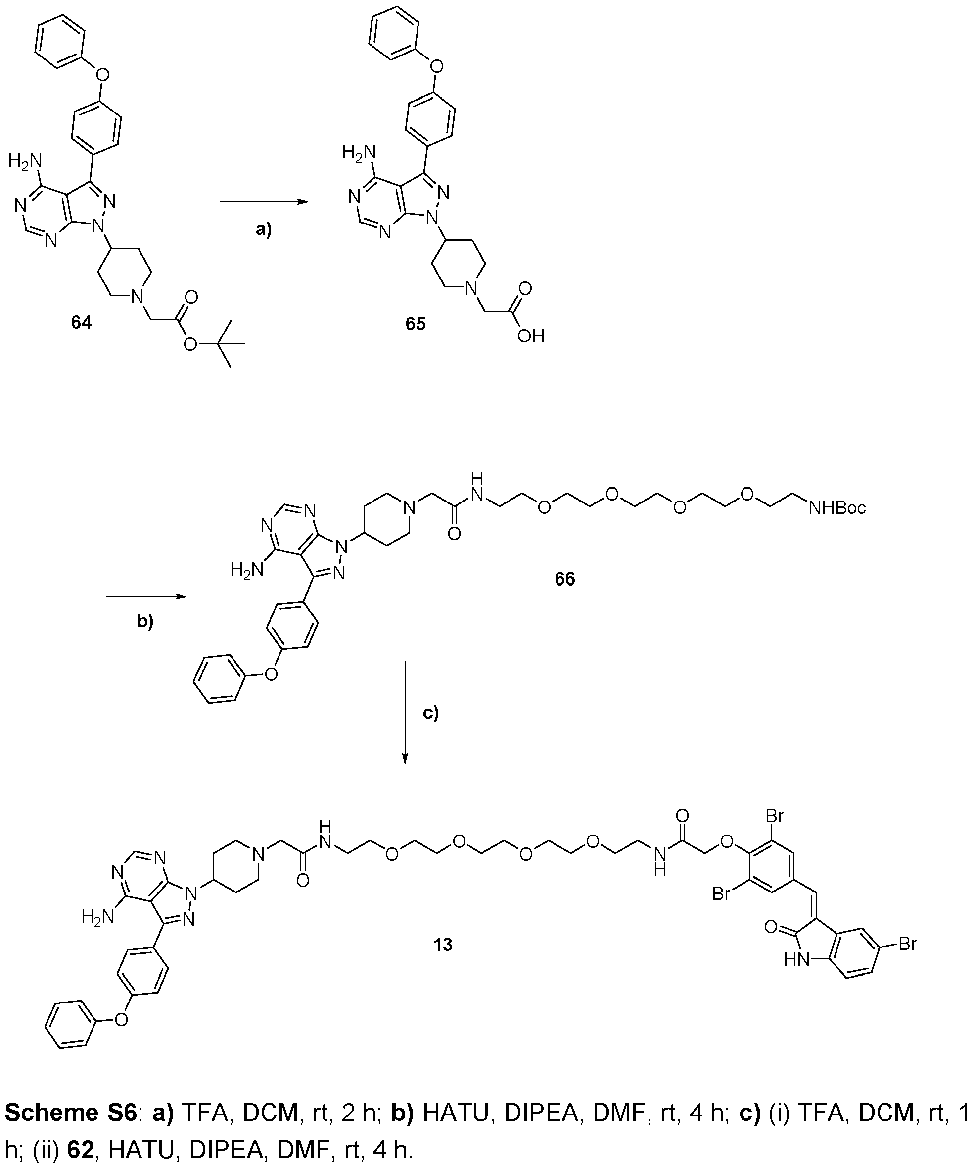

Compound **64** was synthesized according to the literature [Hirst, Gavin C. et al. Preparation of 3-(azahetero)aryl-1H-pyrazolo[3,4-d]pyrimidin-3-amines as protein kinase inhibitors with antiangiogenic properties. PCT Int. Appl., 2002080926, 17 Oct 2002].
**a):** Compound **64** (100 mg, 0.2 mmol, 1 eq.) was dissolved in DCM (1 mL) and TFA (1 mL). After stirring at room temperature for 2 h, the mixture was evaporated to remove the solvent, affording the intermediate **65** without further purification.
**b):** DIPEA (209 µL, 1.2 mmol, 6 eq.) and HATU (91.2 mg, 0.24 mmol, 1.2 eq.) were added sequentially into the solution of compound **65** in DMF (3 mL). After stirred for 10 min, the solution of *tert*-butyl(14-amino-3,6,9,12-tetraoxatetradecyl)carbamate (80.7 mg, 0.24 mmol, 1.2 eq. mmol) in DMF (2 mL) was added into the reaction mixture. After stirring at room temperature for 4 h, the reaction mixture was purified by flash column chromatography (DCM : MeOH = 10 : 1) to obtain the compound **66** (44.2 mg, 29%) as a slightly yellow solid.

### tert-butyl (1-(4-(4-amino-3-(4-phenoxypheny))-1H-pyrazolo[3,4-d]pyrimidin-1-yl)piperidin-1-yl)-2-oxo-6,9,12,15-tetraoxa-3-azaheptadecan-17-yl)carbamate (66) ¹H NMR (500 MHz, CDCl₃) δ 8.28 (s, 1H), 7.61 - 7.50 (m, 3H), 7.35 - 7.29 (m, 2H), 7.12 - 7.06 (m, 3H), 7.03 - 6.99 (m, 2H), 3.58 - 3.49 (m, 15H), 3.44 (td, J = 5.3, 3.0 Hz, 4H), 3.23 - 3.02 (m, 6H), 2.59 - 2.36 (m, 4H), 2.08 - 1.96 (m, 2H), 1.35 (s, 9H).

**¹³C NMR** (126 MHz, CDCl₃) δ 158.59, 157.93, 156.43, 155.56, 153.99, 143.81, 130.08, 127.93, 124.16, 119.61, 119.20, 98.70, 79.30, 70.55, 70.51, 70.49, 70.32, 70.18, 70.07, 61.11, 53.23, 40.41, 38.96, 31.14, 28.52.

**HRMS** calculated for C₃₉H₅₅N₈O₈ [M + H]⁺: 763.4137, found 763.4151.

**c):** Compound **61** (29.4 mg, 0.05 mmol, 1 eq.) was dissolved in DCM (1 mL) and TFA (1 mL). After stirring at room temperature for 2 h, the mixture was evaporated to remove the solvent, affording the intermediate **62** without further purification.

Compound **66** (38.1 mg, 0.05 mmol, 1 eq.) was added into a solution of TFA (1 mL) with DCM (1 mL). After stirring at room temperature for 1 h, the mixture was evaporated to remove the solvent. DMF (3 mL), intermediate **62,** DIPEA (52.3 µL, 0.3 mmol, 6 eq.) and HATU (22.8 mg, 0.06 mmol, 1.2 eq.) were added sequentially to the residue. After stirring at room temperature for 4 h, the reaction mixture was diluted with EtOAc, washed with water and brine, dried over Na₂SO₄, and concentrated in vacuo. Trifluoroacetic acid salt of **13** (12.3 mg, 19%) was obtained as a yellow solid by preparative HPLC with mobile phase of water and MeCN (0.1% TFA).

### (Z)-2-(4-(4-amino-3-(4-phenoxyphenyl)-1H-pyrazolo[3,4-d]pyrimidin-1-yl)piperidin-1-yl)-N-(1-(2.6-dibromo-4-((5-bromo-2-oxoindolin-3-ylidene)methyl)phenoxy)-2 oxo-6,9,12,15-tetr aoxa-3-azaheptadecan-17-yl)acetamide (13)

**¹H NMR** (600 MHz, DMSO-*d*₆) δ 10.87 (s, 1H), 8.79 (s, 2H), 8.69 (t, *J* = 5.7 Hz, 1H), 8.36 (s, 1H), 8.11 (s, 1H), 7.91 (d, *J* = 2.0 Hz, 1H), 7.88 (s, 1H), 7.66 (d, *J* = 8.2 Hz, 2H), 7.47 - 7.43 (m, 2H), 7.41 (dd, *J* = 8.2, 2.0 Hz, 1H), 7.22 - 7.16 (m, 3H), 7.15 - 7.12 (m, 2H), 6.81 (d, *J* = 8.2 Hz, 1H), 5.10 - 5.00 (m, 1H), 4.47 (s, 2H), 3.97 (s, 2H), 3.64 (d, *J =* 11.8 Hz, 2H), 3.57 - 3.47 (m, 17H), 3.42 - 3.29 (m, 6H), 2.64 - 2.54 (m, 1H), 2.34 - 2.12 (m, 2H).

**¹³C NMR** (151 MHz, DMSO-*d*₆) δ 166.67, 166.39, 164.03, 158.60, 158.37, 158.14, 157.92, 157.43, 156.72, 156.15, 153.25, 152.81, 144.19, 140.14, 136.18, 134.60, 133.24, 131.83, 130.17, 130.09, 127.31, 127.29, 126.74, 123.93, 122.92, 119.11, 118.95, 117.23, 117.01, 115.27, 113.32, 113.26, 111.57, 97.33, 71.01, 69.83, 69.80, 69.79, 69.76, 69.61, 68.78, 68.70, 56.60, 51.64, 50.76, 40.06, 38.85, 38.29, 28.06. **HRMS** calculated for C₅₁H₅₅Br₃N₉O₉ [M + H]⁺: 1174.1667, found 1174.1705.

### Example A-5: Preparation of (Z)-N-(2-(2-(2-(2-(2,6-dibromo-4-((5-bromo-2-oxoindolin-3-ylidene)methyl)phenoxy) ethoxy)ethoxy)ethoxy)ethyl)-3-(5,5-difluoro-7,9-dimethyl-5H-dipyrrolo[1,2-c:2',1'-f] [1,3,2]diazaborinin-3-yl)propanamide (Ref.1)

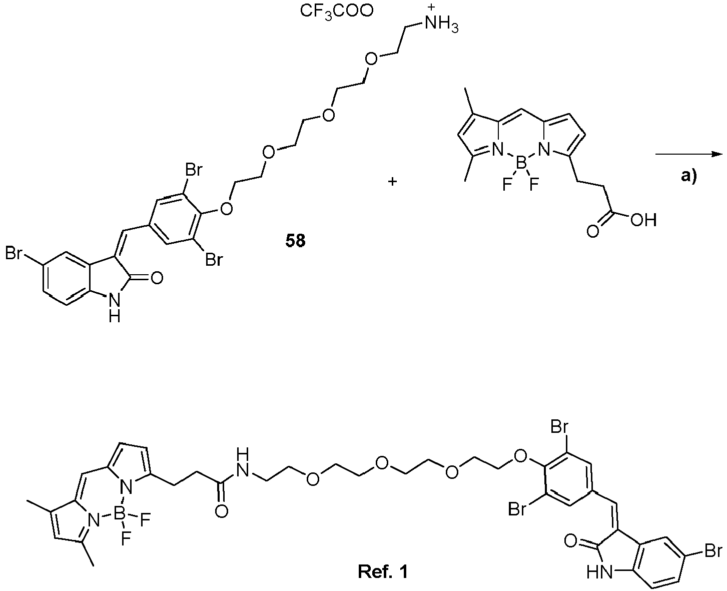

### Scheme S7: a) HATU, DIPEA, DMF, rt, 4 h.

Compound **57** (15.4 mg, 0.02 mmol, 1.2 eq.) was added into a solution of TFA (1 mL) with DCM (1 mL). After stirring at room temperature for 1 h, the mixture was evaporated to remove the solvent, affording the amine intermediate **58** without further purification. The mixture of 3-Bodipy-propanoic acid (5 mg, 0.017 mmol, 1 eq.), DIPEA (17.8 µL, 0.1 mmol, 6 eq.), and HATU (7.6 mg, 0.02 mmol, 1.2 eq.) in DMF (2 mL) was stirred for 10 min. After that, intermediate **58** was added into the resulting solution and stirred at room temperature for 4 h. The reaction mixture was diluted with EtOAc, washed with water and brine, dried over Na₂SO₄, and concentrated in vacuo. Trifluoroacetic acid salt of **Ref.1** (21.8 mg, 38%) was obtained as a yellow solid by preparative HPLC with mobile phase of water and MeCN (0.1 % TFA).

**¹H NMR** (700 MHz, DMSO-*d*₆) δ 10.85 (s, 1H), 8.78 (s, 2H), 7.99 (t, *J* = 5.7 Hz, 1H), 7.91 (d, *J* = 2.0 Hz, 1H), 7.87 (s, 1H), 7.66 (s, 1H), 7.40 (dd, *J* = 8.2, 2.0 Hz, 1H), 7.07 (d, *J* = 4.0 Hz, 1H), 6.81 (d, *J* = 8.2 Hz, 1H), 6.34 (d, *J* = 4.0 Hz, 1H), 6.28 (s, 1H), 4.19 - 4.16 (m, 2H), 3.85 - 3.80 (m, 2H), 3.62 (dd, *J* = 5.9, 3.8 Hz, 2H), 3.57 - 3.50 (m, 6H), 3.42 (t, *J* = 5.8 Hz, 2H), 3.22 (q, *J* = 5.8 Hz, 2H), 3.07 (t, *J* = 7.7 Hz, 2H), 2.50 - 2.47 (m, 2H), 2.46 (s, 3H), 2.25 (s, 3H).

**¹³C NMR** (176 MHz, DMSO-*d*₆) δ 170.90, 166.70, 159.07, 157.85, 154.17, 144.01, 140.06, 136.21, 134.87, 134.41, 132.96, 132.65, 131.70, 128.91, 126.90, 126.83, 125.29, 122.87, 120.23, 117.23, 116.59, 113.23, 111.51, 72.83, 69.94, 69.80, 69.60, 69.49, 69.12, 38.63, 33.64, 23.97, 14.50, 10.99.

**HRMS** calculated for C₃₇H₃₉BBr₃F₂N₄O₆ [M + H]⁺: 921.0475, found 921.0496.

### Example A-6: Preparation of compounds 32, and 39-46.

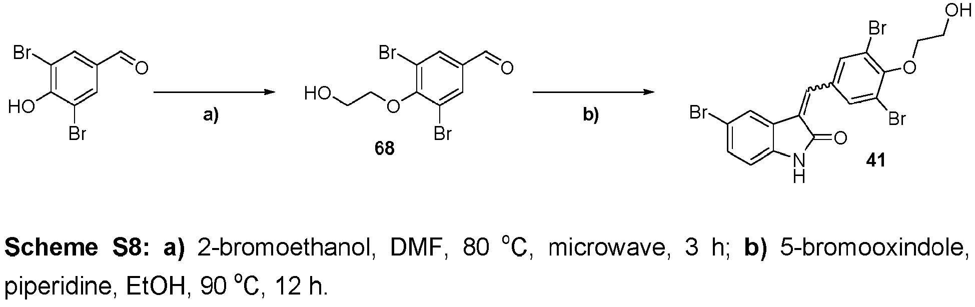

**a):** To a 25 mL flask, 3,5-dibromo-4-hydroxybenzaldehyde (1.68 g, 6 mmol, 1 eq.), 2-bromoethanol (850 µL, 12 mmol, 2 eq.), K₂CO₃ (2.4876 g, 18 mmol, 3 eq.) and anhydrous DMF (8 mL) were added. The resulting mixture was stirred at 80 °C under microwave for 3 h. After cooled to room temperature, the residue was dissolved in EtOAc, washed successively with water and brine. The organic layer was combined, dried over anhydrous Na₂SO₄, and concentrated under reduced pressure. The crude product was subjected to flash chromatography (pentane : EtOAc = 5 : 1). The final product **68** (1.108 g, 57%) was obtained as a white solid.

### 3,5-dibromo-4-(2-hydroxyethoxy)benzaldehyde (68)

**¹H NMR** (500 MHz, CDCl₃) δ 9.88 (s, 1H), 8.06 (s, 2H), 4.33 - 4.27 (m, 2H), 4.07 - 4.01 (m, 2H).

**¹³C NMR** (126 MHz, CDCl₃) δ 188.45, 157.83, 134.14, 131.09, 119.33, 75.44, 62.12. **HRMS** calculated for C₉H₉Br₂O₃ [M + H]⁺: 322.8913, found 322.8917.

**b):** Piperidine (2 µL, 0.02 mmol, 0.1 eq.) were added into the solution of 5-bromooxindole (42.4 mg, 0.2 mmol, 1 eq.) and compound **68** (71.3 mg, 0.22 mmol, 1.1 eq.) in EtOH (3 mL) and stirred for 12 h at 90 °C. The reaction solution was cooled to room temperature, filtered, and the filter cake was washed with cold MeOH (5 mL) for two times to provide **41** as a deep yellow solid (35.2 mg, 34%).

### 5-bromo-3-(3,5-dibromo-4-(2-hydroxyethoxy)benzylidene)indolin-2-one (41)

**¹H NMR** (700 MHz, DMSO-*d*₆) δ 10.85 (s, 1H), 8.78 (s, 2H), 7.91 (d, *J* = 2.0 Hz, 1H), 7.87 (s, 1H), 7.40 (dd, *J* = 8.2, 2.0 Hz, 1H), 6.80 (d, *J* = 8.2 Hz, 1H), 4.94 (t, *J* = 5.7 Hz, 1H), 4.07 (t, *J* = 5.4 Hz, 2H), 3.81 (q, *J* = 5.5 Hz, 2H).

**¹³C NMR** (176 MHz, DMSO-*d*₆) δ 166.69, 154.25, 140.05, 136.22, 134.87, 132.61, 131.67, 126.88, 126.82, 122.86, 117.27, 113.22, 111.49, 75.11, 60.10.

**HRMS** calculated for C₁₇H₁₃Br₃NO₃ [M + H]⁺: 515.8440, found 515.8436.

### 5,6-dichloro-3-(3,5-dibromo-4-(2-hvdroxvethoxv)benzvlidene)indolin-2-one (32)

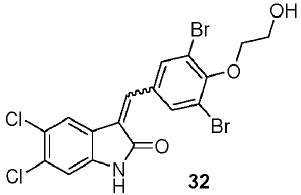

Compound **32** (29.5 mg, 29%) was obtained as a yellow solid.

**¹H NMR** (500 MHz, DMSO-*d*₆) δ 10.95 (s, 1H), 8.75 (s, 1.09H), 8.03 (s, 0.92H), 7.93 (d, *J* = 28.4 Hz, 1.14H), 7.63 (s, 0.46H), 7.54 (s, 0.52H), 7.07 (s, 0.47H), 7.01 (s, 0.56H), 4.94 (t, *J* = 5.6 Hz, 1H), 4.14 - 4.03 (m, 2H), 3.90 - 3.76 (m, 2H).

**¹³C NMR** (126 MHz, DMSO-*d*₆) δ 167.86, 166.68, 154.41, 153.88, 143.07, 140.61, 136.26, 135.60, 134.68, 133.53, 132.70, 132.43, 132.24, 131.07, 127.24, 126.06, 125.34, 123.62, 123.47, 122.92, 121.81, 121.11, 118.04, 117.32, 111.78, 111.13, 75.13, 75.10, 60.10.

**HRMS** calculated for C₁₇H₁₂Br₂Cl₂NO₃ [M + H]⁺: 505.8556, found 505.8561.

### 1-acetyl-5-bromo-3-(3,5-dibromo-4-(2-hydroxyethoxy)benzylidene)indolin-2-one (39)

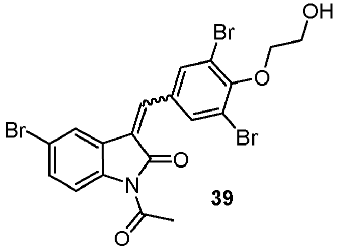

Compound **39** (19.0 mg, 17%) was obtained as a yellow solid.

**¹H NMR** (600 MHz, DMSO-*d*₆) δ 8.60 (s, 1.35H), 8.13 (d, *J* = 8.7 Hz, 0.32H), 8.06 (d, *J* = 2.1 Hz, 0.68H), 8.05 - 7.96 (m, 2H), 7.78 (s, 0.31H), 7.66 (d, *J* = 2.1 Hz, 0.31H), 7.59 (dd, *J* = 8.8, 2.1 Hz, 0.31H), 7.53 (dd, *J* = 8.7, 2.1 Hz, 0.68H), 5.00 - 4.89 (m, 1H), 4.16 - 4.03 (m, 2H), 3.88 - 3.76 (m, 2H), 2.62 (s, 3H).

**¹³C NMR** (151 MHz, DMSO-*d*₆) δ 170.56, 170.32, 166.59, 165.16, 154.68, 154.17, 139.35, 137.45, 136.76, 136.35, 133.55, 132.90, 132.27, 132.04, 131.83, 126.84, 125.70, 124.34, 124.29, 123.27, 122.49, 118.08, 118.04, 117.61, 117.37, 117.08, 116.47, 75.17, 75.14, 60.11, 60.09, 26.64, 26.48.

**HRMS** calculated for C₁₉H₁₅Br₃NO₄ [M + H]⁺: 557.8546, found 557.8540.

### 3-(3,5-dibromo-4-(2-hydroxyethoxy)benzylidene)-5-nitroindolin-2-one (40)

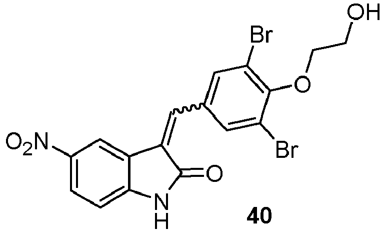

Compound **40** (28.1 mg, 29%) was obtained as a yellow solid.

**¹H NMR** (600 MHz, DMSO-*d*₆) δ 11.41 (s, 1H), 8.82 (s, 2H), 8.63 (d, *J* = 2.3 Hz, 1H), 8.17 (dd, *J* = 8.6, 2.3 Hz, 1H), 8.13 (s, 1H), 7.01 (d, *J* = 8.6 Hz, 1H), 4.94 (t, *J* = 5.7 Hz, 1H), 4.08 (t, *J* = 5.4 Hz, 2H), 3.82 (q, *J* = 5.4 Hz, 2H).

**¹³C NMR** (151 MHz, DMSO-*d*₆) δ 167.30, 154.62, 146.35, 142.14, 136.69, 136.49, 132.37, 125.84, 125.73, 125.26, 117.35, 115.84, 109.63, 75.14, 60.11.

**HRMS** calculated for C₁₇H₁₃Br₂N₂O₅ [M + H]⁺: 482.9186, found 482.9196.

### 4-bromo-3-(3,5-dibromo-4-(2-hydroxyethoxy)benzylidene)indolin-2-one (42)

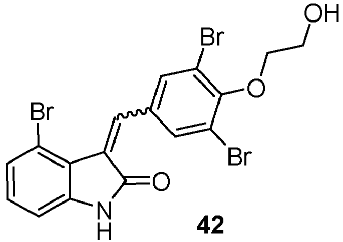

Compound **42** (35.2 mg, 34%) was obtained as a yellow solid.

**¹H NMR** (500 MHz, DMSO-*d*₆) δ 10.91 (s, 1H), 8.46 (s, 2H), 8.45 (s, 1H), 7.25 - 7.14 (m, 2H), 6.88 (d, *J* = 7.6 Hz, 1H), 4.94 (t, *J* = 5.7 Hz, 1H), 4.06 (t, *J* = 5.4 Hz, 2H), 3.82 (q, *J* = 5.5 Hz, 2H).

**¹³C NMR** (126 MHz, DMSO-*d*₆) δ 166.11, 153.80, 143.58, 136.00, 135.82, 132.08, 130.74, 128.09, 126.43, 120.59, 116.89, 116.49, 109.09, 75.02, 60.08.

**HRMS** calculated for C₁₇H₁₃Br₃NO₃ [M + H]⁺: 515.8440, found 515.8436.

### 7-bromo-3-(3,5-dibromo-4-(2-hydroxyethoxy)benzylidene)indolin-2-one (43)

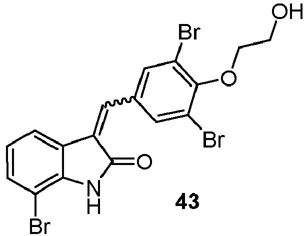

Compound **43** (59.0 mg, 57%) was obtained as a yellow solid.

**¹H NMR** (500 MHz, DMSO-*d*₆) δ 10.98 (s, 1H), 8.78 (s, 1.25H), 7.99 (s, 0.73H), 7.83 (s, 0.65H), 7.69 (d, *J* = 7.6 Hz, 0.66H), 7.60 (s, 0.42H), 7.50 - 7.37 (m, 1.41H), 6.99 (t, *J* = 7.8 Hz, 0.62H), 6.87 (t, *J* = 7.9 Hz, 0.38H), 4.99 - 4.90 (m, 1H), 4.08 (q, *J* = 5.4 Hz, 2H), 3.89 - 3.76 (m, 2H).

**¹³C NMR** (126 MHz, DMSO-*d*₆) δ 168.03, 166.90, 154.23, 153.69, 140.06, 136.17, 134.86, 134.25, 133.43, 133.14, 132.97, 132.53, 131.94, 128.71, 127.63, 126.30, 122.85, 122.38, 121.21, 119.10, 117.97, 117.24, 102.93, 102.04, 75.11, 75.06, 60.10. **HRMS** calculated for C₁₇H₁₃Br₃NO₃ [M + H]⁺: 515.8440, found 515.8438.

### 5-bromo-3-(3,5-dibromo-4-(2-hydroxyethoxy)benzylidene)-1-methylindolin-2-one (44)

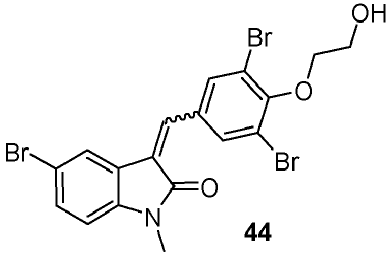

Compound **44** (20.2 mg, 19%) was obtained as a yellow solid.

**¹H NMR** (600 MHz, DMSO-*d*₆) δ 8.79 (s, 1.51H), 8.01 (s, 0.48H), 7.93 (d, *J* = 2.0 Hz, 0.75H), 7.89 (s, 0.75H), 7.67 (s, 0.23H), 7.56 - 7.52 (m, 0.48H), 7.48 (dd, *J* = 8.3, 1.9 Hz, 0.75H), 7.04 (d, *J* = 8.8 Hz, 0.22H), 6.98 (d, *J* = 8.3 Hz, 0.75H), 4.98 - 4.91 (m, 1H), 4.12 - 4.04 (m, 2H), 3.87 - 3.79 (m, 2H), 3.19 (s, 3H).

**¹³C NMR** (151 MHz, DMSO-*d*₆) δ 166.38, 164.86, 154.35, 153.82, 143.47, 141.18, 136.28, 135.06, 134.59, 133.55, 132.77, 132.76, 132.46, 131.58, 127.03, 125.67, 124.55, 122.50, 121.86, 117.97, 117.28, 113.88, 113.33, 111.07, 110.51, 75.12, 75.10, 60.10, 60.09, 26.15, 26.04.

**HRMS** calculated for C₁₈H₁₅Br₃NO₃ [M + H]⁺: 529.8597, found 529.8594.

### 6-bromo-3-(3,5-dibromo-4-(2-hydroxyethoxy)benzylidene)indolin-2-one (45)

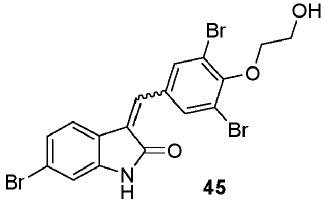

Compound **45** (58.0 mg, 56%) was obtained as a yellow solid.

**¹H NMR** (500 MHz, DMSO-*d*₆) δ 10.79 (s, 1H), 7.98 (s, 2H), 7.58 (s, 1H), 7.33 (d, *J* = 8.3 Hz, 1H), 7.13 - 7.08 (m, 1H), 7.04 (d, *J* = 1.8 Hz, 1H), 4.94 (t, *J* = 5.7 Hz, 1H), 4.08 (t, *J* = 5.4 Hz, 2H), 3.82 (q, *J* = 5.5 Hz, 2H).

**¹³C NMR** (126 MHz, DMSO-*d*₆) δ 168.04, 153.62, 144.66, 133.35, 133.24, 133.17, 128.00, 123.96, 123.74, 123.18, 119.77, 118.01, 113.10, 75.04, 60.09.

**HRMS** calculated for C₁₇H₁₃Br₃NO₃ [M + H]⁺: 515.8440, found 515.8437.

### 3-(3,5-dibromo-4-(2-hydroxyethoxy)benzylidene)-1-phenylindolin-2-one (46)

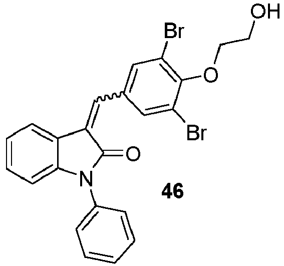

Compound **46** (23.7 mg, 23%) was obtained as a yellow solid.

**¹H NMR** (600 MHz, DMSO-*d*₆) δ 8.78 (s, 0.60H), 8.06 (s, 1.35H), 7.95 (s, 0.28H), 7.83 (dd, *J* = 7.7, 1.2 Hz, 0.29H), 7.72 (s, 0.66H), 7.63 - 7.58 (m, 2H), 7.56 (dd, *J* = 7.8, 1.1 Hz, 0.76H), 7.52 - 7.46 (m, 3H), 7.33 - 7.27 (m, 1H), 7.16 (t, *J* = 7.5 Hz, 0.31H), 7.03 (t, *J* = 7.3 Hz, 0.67H), 6.79 (d, *J* = 7.9 Hz, 0.70H), 6.75 (d, *J* = 7.8 Hz, 0.30H), 4.14 - 4.05 (m, 2H), 3.87 - 3.79 (m, 2H).

**¹³C NMR** (151 MHz, DMSO-*d*₆) δ 166.32, 164.76, 154.12, 153.69, 144.04, 141.95, 136.12, 134.25, 134.16, 134.09, 133.81, 133.75, 133.44, 133.20, 132.64, 130.95, 130.72, 129.68, 129.57, 128.17, 128.10, 127.69, 126.95, 126.86, 126.60, 123.62, 122.51, 122.35, 120.23, 119.99, 118.00, 117.22, 109.62, 108.98, 75.11, 75.09, 60.12, 60.09.

**HRMS** calculated for C₂₃H₁₈Br₂NO₃ [M + H]⁺: 513.9648, found 513.9654.

### Biological Methods

### Methods:

**Reagents and antibodies:** Following reagents were used: Chloroquine (Sigma, C6628), NH₄Cl (J.T.Baker, 0018), and Bafilomycin A1 (Cell Signaling Technology (CST), 54645S), Carfilzomib (Abcam, ab216469), MG-132 (CST, 2194S), MLN4924 (CST, 85923S), and JQ1 (Sigma-Aldrich, SML1524). Deltazinone is from COMAS. Compounds **a1-a21** were purchased from ChemDiv. Details of chemical syntheses and NMR spectrum of other compounds can be found in the Supplementary Information as Supplementary Scheme S1-11. The antibodies for BRD2 (5848S), BRD4 (13440S), LC3B (2775S), GAPDH (2118S), DDB1 (6998S), BTK (8547S) and BLK (3262S) were purchased from Cell Signaling Technology. The antibodies for BRD2 (for In-Cell Western, ab139690), BRD3 (ab50818), and RBX1 (ab133565) were purchased from Abcam. The antibody for PDEδ (PA5-22008) was purchased from Invitrogen. The antibody for Vinculin (V9131) was purchased from Sigma-Aldrich, and the antibody for DCAF11 (A15519) was purchased from ABclonal. The HRP secondary antibodies (31460 for anti-rabbit and 62-6520 for anti-mouse) were purchased from Invitrogen. APC anti-CD90.1/Thy1.1 (202526, BioLegend) and Human TruStain FcX^{™} Fc Receptor Blocking Solution (422302, BioLegend) were used for FACS-based CRISPR/Cas9 screens.

### Cell culture

All mammalian cells were cultured under sterile conditions in a humidified atmosphere at 5% CO₂ at 37°C. Jurkat and Ramos cells were cultured in RPMI1640 medium with 10% FBS. HEK293T, Lenti-X 293T lentiviral packaging cells, MDA-MB-231 and Hep G2 cells were cultured in DMEM medium with 10% FBS, 1 mM sodium pyruvate and nonessential amino acids. KBM7 and HAP1 cells were cultured in IMDM medium with 10% FBS. All cell lines were regularly tested for mycoplasma contamination and were always free of mycoplasma.

### Western Blot

Cells were washed with PBS and lysed in lysis buffer (50 mM Tris-HCl, pH 7.4 , 150 mM NaCl , 1% Triton X-100 , 1% sodium deoxycholate , 0.1% SDS) with complete protease inhibitor cocktail (Roche, 11836170001) for 30 min on ice. For Ramos cells, cell pellets were lysed in lysis buffer (50 mM Tris-HCl, pH 8 , 150 mM NaCl , 1% NP-40, 0.5% sodium deoxycholate) with complete protease inhibitor cocktail. After centrifugation at 16,000 x g, the supernatant was transferred into a new tube and the protein concentration was determined by DC protein assay (BIO-RAD, 5000116). Proteins were separated by SDS-PAGE and transferred to a PVDF membrane (Thermo scientific, 0.45 µm, 88518). For PDE6, BRD2, GAPDH, BRD4, Vinculin and BTK, the membranes were blocked with Intercept^{®} Blocking Buffer (LI-COR Biosciences, 927-70001) at room temperature for 1 h. The primary antibodies for these proteins were diluted with Intercept^{®} Blocking Buffer (1:500 for PDEδ, 1:3000 for Vinculin, and 1:1000 for other proteins) and incubated with the membranes overnight at 4 °C. Membranes were washed with PBS+0.1% Tween 20 (PBS-T) five times and incubated with secondary antibodies conjugated to IRDye^{®} Infrared Fluorescent Dyes (1:5000, LI-COR Biosciences) at room temperature for 1 h with protection from light. After washing the membrane, signals were visualized using the ChemiDoc MP Imaging System (BIO-RAD Laboratories). For detection of LC3B, BRD3, BLK, DCAF11, DDB1, and RBX1, the membranes were blocked with 5% milk in PBS-T at room temperature for 1 h. The primary antibodies for these proteins were diluted in 5% milk in PBS-T buffer (1:200 for BRD3, 1:1000 for other proteins) and incubated with the membranes overnight at 4 °C. Membranes were washed with PBS-T five times and incubated with HRP secondary antibodies (1:1500) at room temperature for 1 h. After washing the membrane, protein detection was performed using Western blotting detection reagent (Thermo Scientific, 34095) and the ChemiDoc MP Imaging System (BIO-RAD). Relative band intensities were quantified using Image Lab (BIO-RAD).

### RT-qPCR

Jurkat cells were grown in 12-well plates and treated with compounds for the indicated time. RNA was extracted using the RNeasy Plus Mini Kit (QIAGEN, 74134), and DNA was removed by DNase digestion with RNase-free DNase Set (QIAGEN, 79256). cDNA was synthesized using the QuantiTect Reverse Transcription Kit (QIAGEN, 205313). The mRNA expression level was evaluated with the QuantiFast SYBR Green PCR Kit (Qiagen, 330502), using the CFX96 Real-Time PCR Detection System (BIO-RAD Laboratories), and relative expression levels were calculated using the ΔΔ*C*ₜ method using GAPDH as reference and analyzed in GraphPad Prism 9.0.

The sequences of the primers for *PDE6D* were 5'-GCAGTCCTTGATAGAGGCAGCA-3' (forward) (SEQ ID NO: 1) and 5'-GAAAAGTCTCACTCTGGATGTGC-3' (reverse) (SEQ ID NO: 2).

For *GAPDH,* the sequences were 5'-GTCTCCTCTGACTTCAACAGCG-3' (forward) (SEQ ID NO: 3) and 5'-ACCACCCTGTTGCTGTAGCCAA-3' (reverse) (SEQ ID NO: 4). For *BRD2,* the sequences were 5'-CGGCTTATGTTCTCCAACTGCTA-3' (forward) (SEQ ID NO: 5), 5'-GGCAGTAGAGACTGGTAAAGGC-3' (reverse) (SEQ ID NO: 6).

For *BRD3,* the sequences were 5'-CCAACCATCACTGCAAACGTCAC-3' (forward) (SEQ ID NO: 7), 5'-GGAGTGGTTGTGTCTGCTTTCC-3' (reverse) (SEQ ID NO: 8). For *BRD4,* the sequences were 5'-CGCTATGTCACCTCCTGTTTGC-3' (forward) (SEQ ID NO: 9), 5'-ACTCTGAGGACGAGAAGCCCTT-3' (reverse) (SEQ ID NO: 10).

### RNA interference

Hep G2 cells and MDA-MB-231 cells were seeded in 6-well plates and non-targeting siRNA (Horizon Discovery Ltd, D-001810-10-05), DCAF11 siRNA (Horizon Discovery Ltd, SMARTPool, L-019051-01-0005) or RBX1 siRNA (Horizon Discovery Ltd, SMARTPool, L-004087-00-0005) were transfected into the cells with DharmaFECT 1 transfection reagent (Horizon Discovery Ltd, T-2001-02) following the manufacturer's protocol. After incubation with DCAF11 siRNA (50 nM, 72 h) or RBX1 siRNA (30 nM, 48 h), compounds were added and cells were collected and lysed as mentioned above.

### CRISPR-Cas9 knockout screens:

For pooled FACS-based CRISPR-Cas9 BRD4 protein stability screens, a CRL-focused sgRNA library was lentivirally packaged using polyethylenimine (PEI MAX^{®} MW 40,000, Polysciences) transfection of Lenti-X cells and the lentiviral pCMVR8.74 helper (Addgene plasmid # 22036) and pMD2.G envelope (Addgene plasmid # 12259) plasmids. The virus containing supernatant was cleared of cellular debris by filtration through a 0.45-µm PES filter and used to transduce KBM7 BRD4-BFP reporter cells harboring a doxycycline inducible Cas9 allele at a multiplicity of infection (MOI) of 0.05 and 1,000-fold library representation. Library-transduced cells were selected with G418 (1 mg ml-1, Gibco) for 14 days, expanded and Cas9 expression was induced with DOX (0.4µg ml-1, PanReac AppliChem).

3 days after Cas9 induction, 25 million cells per condition were treated with DMSO (1:1000), **10** (1 µM), **9** (0.5 µM) or dBET6 (10 nM, MedChem Express) for 6 hours in two biological replicates. Cells were washed with PBS, stained with Zombie NIR^{™} Fixable Viability Dye (1:1000, BioLegend) and APC anti-mouse CD90.1/Thy-1.1 antibody (1:400, BioLegend) in the presence of Human TruStain FcX^{™} Fc Receptor Blocking Solution (1:400, BioLegend), and fixed with 0.5 mL methanol-free paraformaldehyde 4% (Thermo Scientific^{™} Pierce^{™}) for 30 min at 4 °C, while protected from light. Cells were washed with and stored in FACS buffer (PBS containing 5% FBS and 1 mM EDTA) at 4 °C over night. The next day, cells were strained trough a 35 µm nylon mesh and sorted on a BD FACSAria^{™} Fusion (BD Biosciences) using a 100 µm nozzle. Aggregates, dead (ZombieNIR positive), Cas9-negative (GFP) and sgRNA librarynegative (Thy1.1-APC) cells were excluded, and the remaining cells were sorted based on their BRD4-BFP and mCherry levels into BRD4^{HIGH} (10% of cells), BRD4^{MID} (30%) and BRD4^{LOW} (10%) fractions. For each sample, cells corresponding to at least 2,000-fold library representation were sorted per replicate.

Next-generation sequencing (NGS) libraries of sorted cell fractions were prepared. In brief, genomic DNA was isolated by cell lysis (10 mM Tris-HCl, 150 mM NaCl, 10 mM EDTA, 0.1% SDS), proteinase K treatment (New England Biolabs) and DNAse-free RNAse digest (Thermo Fisher Scientific), followed by two rounds of phenol extraction and 2-propanol precipitation. Isolated genomic DNA was subjected to several freezethaw cycles before nested polymerase chain reaction (PCR) amplification of the sgRNA cassette.

Barcoded NGS libraries for each sorted population were generated using a two-step PCR protocol using AmpliTaq Gold (Invitrogen. The resulting PCR products were purified using Mag-Bind^{®} TotalPure NGS beads (Omega Bio-tek) and amplified in a second PCR introducing the standard Illumina adapters. The final Illumina libraries were bead-purified, pooled and sequenced on a HiSeq 3500 platform (Illumina).

### CRISPR-mediated DCAF11 knockout in KBM7 cell lines:

DCAF11-targeting sgRNAs were lentivirally packaged using PEI transfection of Lenti-X cells. The viral supernatant was harvested after 72 h, cleared of cellular debris by filtration through a 0.45-µm PES filter and stored in aliquots at -80 °C. The appropriate amount of viral supernatant was transduced into KBM7 cells (1 × 10⁶ cells/mL) via spinfection (2000 rpm, 1 h, 37 °C) with polybrene (8 µg/mL). Two days post transduction, the cells were selected with puromycin (1 µg/mL) for a total of 5 days, after which the knockout pools were validated via western blotting.

### Cloning of constructs

The BRD2 cDNA with N-terminal FLAG tag for mammalian cell expression was obtained by inserting the BRD2 cDNA (Horizon Discovery, MHS6278-202759982) into the pOPIN-E vector with the forward primer (5'-AGGAGATATACCATGGATTACAAGGATGACGACGATAAGGGATCTATGCTGCAAAA CGTGACTCCCC-3';) (SEQ ID NO: 11) and reverse primer (5'-GTGATGGTGATGTTTTTAGCCTGAGTCTGAATCACTGGTGTCTG-3') (SEQ ID NO: 12) using the In-Fusion HD Cloning Kit (Takara Clonetech, 638909). The BRD4-BFP reporter was engineered by inserting BRD4 short isoform (amino acids 1-719; Twist Bioscience) and mTagBFP into a modified pRRL lentiviral backbone. DCAF11-targeting sgRNAs were cloned into pLentiCRISPRv2 (Addgene #52961) following standard protocol. The following primer sequences were used: 5'-CACCGAGAGTTGGAGATCAGATACC-3' (forward) (SEQ ID NO: 13), 5'-AAAC GGTATCTGATCTCCAACTCTC-3' (reverse) (SEQ ID NO: 14) for *DCAF11* KO1; 5'-CACCGGATGGACGAGAAGTACTAGG-3' (forward) (SEQ ID NO: 15), 5'-AAACCCTAGTACTTCTCGTCCATCC-3' (reverse) (SEQ ID NO: 16) for *DCAF11* KO2.

### Co-immunoprecipitation

Hep G2 cells were seeded in 6-well plates, 24 h later, cells were transiently transfected with HA-DCAF11 (Sino Biological, HG16257-NY) and FLAG-BRD2 for 48 h using the FuGENE^{®} HD transfection reagent (Promega, E2311). After treatment with MG-132 (10 µM, 40 min) and compound 9 (10 µM, 1.5 h), cells were collected and lysed in lysis buffer (25 mM Tris-HCl pH 7.4, 150 mM NaCl, 10% glycerol, 1% NP-40) with complete protease inhibitor cocktail (Roche) on ice for 30 min, followed by centrifugation at 16,000 × g for 15 min collection of the supernatant. After determination of the protein concentration, cell lysates were incubated with anti-FLAG magnetic beads (30 µl / sample, Sigma, M8823) at 4 °C for 4 h and washed three times with washing buffer (0.2% NP-40, 25 mM Tris-HCl pH 7.4, 150 mM NaCl). The beads were diluted with PBS, mixed with SDS sample buffer and heated at 95 °C for 10 min, followed by western blot analysis.

### In-gel fluorescence

Purified DCAF11 protein (1 µg, Origene, TP760719) was diluted in PBS buffer that is mixed with compound **2** or BODIPY derivative **Ref.1** at the indicated concentration at room temperature. Samples were diluted with 5 × SDS sample loading buffer and heated at 95 °C for 5 min. Proteins were separated using SDS-PAGE and were visualized using the ChemiDoc MP Imaging System (BIO-RAD) at 488 nm and the results were analyzed by Image Lab (BIORAD).

### In-Cell Western

Hep G2 cells were seeded in 96-well plates and incubated for 24 h. Compounds were added for the indicated time at the indicated concentration. Cells were then washed once with PBS and fixed with 4% paraformaldehyde for 30 min at room temperature. The fixed cells were permeabilized with 0.5 % Triton-X100 for 15 min followed by block with Intercept^{®} Blocking Buffer (LI-COR Biosciences, 927-70001) for 1 h at room temperature. Primary antibodies for BRD2 (1:800) and Vinculin (1:2000) were diluted with LI-COR buffer and incubated with the cells at 4 °C overnight. Cells were washed with PBS-T, and the secondary antibodies conjugated to IRDye (1:1000, LI-COR Biosciences) were added to the cells for 1 h at room temperature. After washing the cells, the signal was recorded with the Odyssey^{®} CLx Infrared Imaging System (LI-COR Biosciences) and the protein levels were analyzed by Image Studio (LI-COR Biosciences). BRD2 protein levels were normalized to the levels of reference protein vinculin.

### Cell viability assay

Jurkat cells (3 × 10³) and Hep G2 cells (3.5 × 10³) were seeded in 96-well plates in 100 µL culture medium. Cells were treated with compounds dissolved in 50 µl medium at the indicated concentration for 72 h, at the same time, one plate of cells without compounds treatment was used for the cell count at time zero. After adding 40 µl of CellTiter-Glo^{®} reagent (Promega, G7571) to each well for 10 min at room temperature, the luminescence was measured on a Spark^{®} Multimode Microplate Reader (Tecan). The data was analyzed by the GraphPad Prism 9.0.

### Caspase-3/7 Activity Assay

Jurkat cells (3 × 10³) were seeded in 96-well plates in 25 µl culture medium. Cells were treated with compounds dissolved in 25 µl medium at the indicated concentration for 18 h. After adding 50 µl of Caspase-Glo^{®} 3/7 Reagent (Promega, G8091) to each well for 30 min at room temperature, the luminescence was measured on a Spark^{®} Multimode Microplate Reader (Tecan). The data was analyzed by the GraphPad Prism 9.0.

### Example B-1: qualitative ICW assay

The enzymatic activity of the β5 subunit of the human constitutive proteasome (CPS) is analyzed. In another assay, which differs from the assay of the human constitutive proteasome only in using the human immunoproteasome (IPS) the enzymatic activity of the β5 subunit of the human immunoproteasome (IPS) is analyzed.

Incubation of the enzyme with its fluorogenic substrate Suc-LLVY-AMC leads to liberation of the fluorescent dye AMC which can be measured with a suitable plate reader.

**Table S3. Activity of compounds 2, a1-a21, 39-46, Ref.2 and Ref.3 by qualitative ICW assay.**

| **Comp** | **Structure** | **Value (Mean** ± **SD)** | **Comp** | **Structure** | **Value (Mean ± SD)** |
|---|---|---|---|---|---|
| **2** | 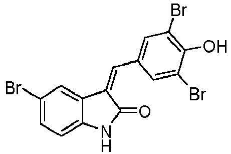 | 44.85 ± 4.23 | **a16** | 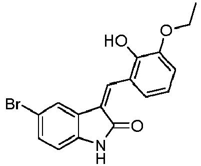 | 38.78 ± 7.19 |
| **a1** | 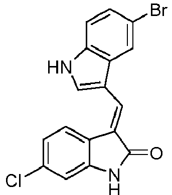 | 22.45 ± 2.48 | **a17** | 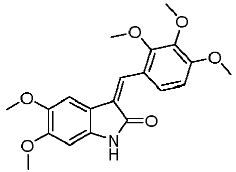 | 43.80 ± 10.34 |
| **a2** | 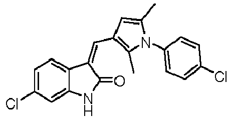 | 23.05 ± 3.61 | **a18** | 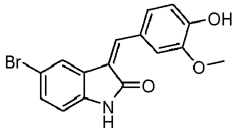 | 44.21 ± 2.82 |
| **a3** | 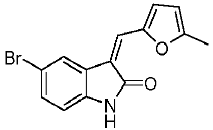 | 27.96 ± 5.89 | **a19** | 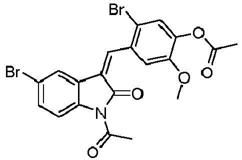 | 46.27 ± 6.95 |
| **a4** | 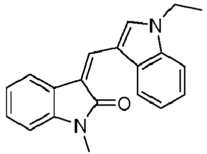 | 28.31 ± 1.06 | **a20** | 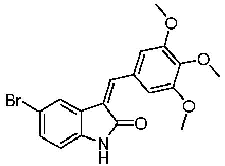 | 46.38 ± 2.20 |
| **a5** | 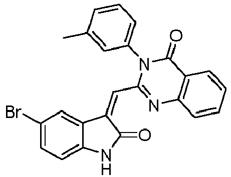 | 28.93 ± 2.95 | **a21** | 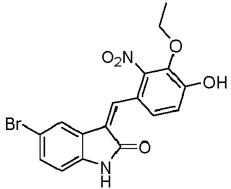 | 46.41 ± 4.18 |
| **a6** | 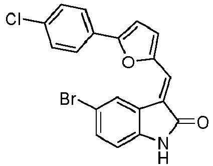 | 32.30 ± 3.56 | **39** | 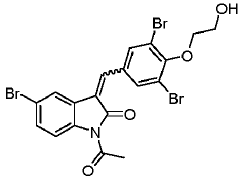 | 49.83 ± 3.84 |
| **a7** | 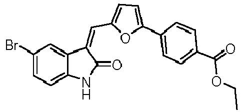 | 32.83 ± 1.96 | **40** | 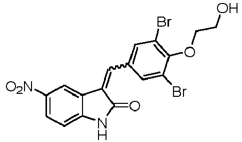 | 51.57 ± 11.55 |
| **a8** | 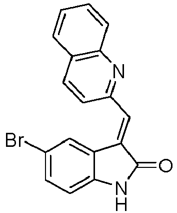 | 38.97 ± 1.11 | **41** | 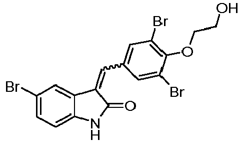 | 55.54 ± 6.61 |
| **a9** | 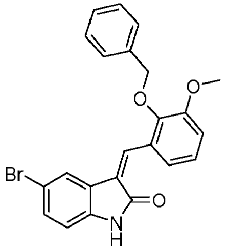 | 25.66 ± 3.60 | **42** | 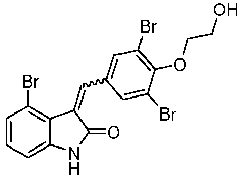 | 55.89 ± 8.40 |
| **a10** | 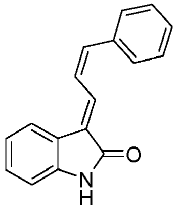 | 27.19 ± 3.29 | **43** | 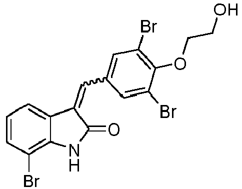 | 57.04 ± 9.16 |
| **a11** | 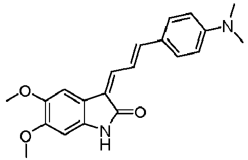 | 31.86 ± 2.35 | **44** | 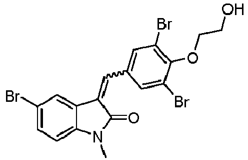 | 67.69 ± 4.30 |
| **a12** | 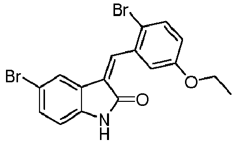 | 36.18 ± 4.38 | **45** | 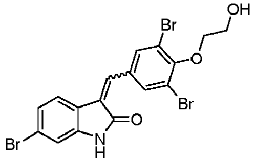 | 70.23 ± 9.63 |
| **a13** | 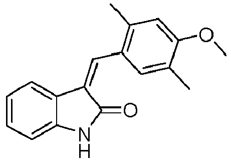 | 36.20 ± 1.35 | **46** | 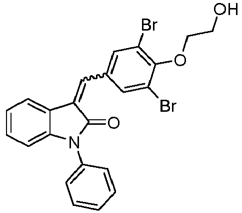 | 72.76 ± 4.44 |
| **a14** | 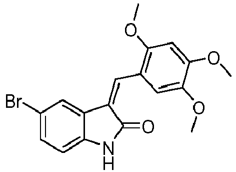 | 38.22 ± 2.33 | **Ref.2** | 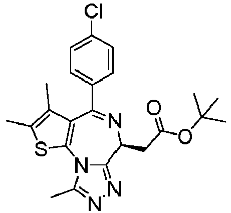 | 67.81 ± 1.42 |
| **a15** | 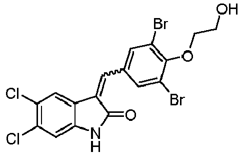 | 38.55 ± 3.55 | **Ref.3** | 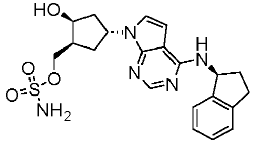 | 112.98 ± 3.16 |

| | | | | | |
|---|---|---|---|---|---|
| Value: Relative BRD2 levels remaining in Hep G2 cells after treatment with compounds (**2**, **a1-a21**, **39-46, Ref.2.** - **Ref.3)** followed by treatment with **9**, with DMSO control for normalization. | | | | | |

## Claims

1. A compound of the general formula (I): 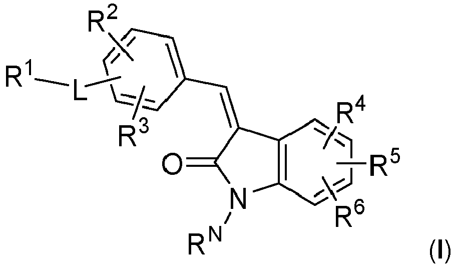 wherein
**L** represents **-L₃-L₂-L₁-**;
**L₁** represents a covalent bond, -O-, -NH-, or -NH-CO-CH₂-O-;
**L₂** represents -(C₂H₄)ₙ-, -CH₂-(C₂H₄)ₙ-, -(C₂H₄O)ₙ-, -(CH₂)ₘ-(C₂H₄O)ₙ-, -(C₂H₄O)ₙ-(CH₂)ₘ- -(CH₂)ₘ-(OC₂H₄)ₙ-, -(OC₂H₄)ₙ-(CH₂)ₘ-, -(CH₂)ₘ-(C₂H₄O)ₙ-(CH₂)ₒ-, -(CH₂)ₘ-(OC₂H₄)ₙ-(CH₂)ₒ-, or -(CH₂)ₘ-(OC₂H₄)ₙ-O-(CH₂)ₒ-;
**L₃** represents a covalent bond, -O-, -NH-, -NH-CO-, -CO-NH-, -CH₂-CO-NH-, or -NH-CO-NH-;
**n** is an integer selected from 1, 2, 3, 4, 5, 6, 7, 8, 9, and 10;
**m** and **o** are independently of each other an integer selected from 1, 2, 3, and 4;
**R¹** represents 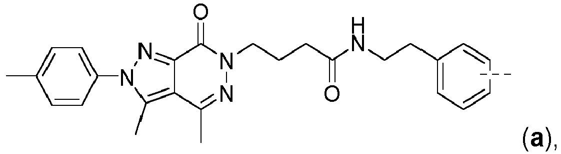 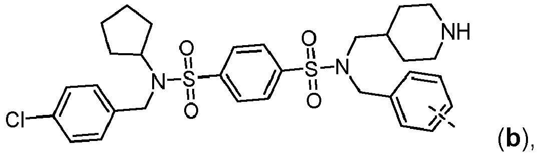 or 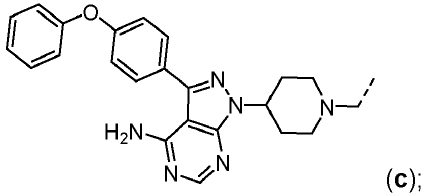
**R², R³, R⁴, R⁵,** and **R⁶** represent independently of each other -H, -F, -Br, -Cl, -I, -OH, -CN, -NO₂, -CH₃, -C₂H₅, -C₃H₇, -CH(CH₃)₂, -C₄H₉, -CH₂-CH(CH₃)₂, -cyclo-C₃H₅, -cyclo-C₄H₇, -cyclo-C₅H₉, -cyclo-C₆H₁₁, -OCH₃, -OC₂H₅, -OCOCH₃, -OC₃H₇, -O-cyclo-C₃H₅, -OCH(CH₃)₂, -OC(CH₃)₃, -OC₄H₉, -OPh, -OCH₂-Ph, -OCH₂F, -OCHF₂, -OCF₃, -CH₂F, -CHF₂, -CF₃, -CH₂-CH₂F, -CH₂-CHF₂, -CH₂-CF₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO2C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, , -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH=CH₂, -CH₂-CH=CH₂, -C(CH₃)=CH₂, -CH=CH-CH₃, -CH=CH-Ph, -C≡CH, -C≡C-CH₃, -CH₂-C≡CH, 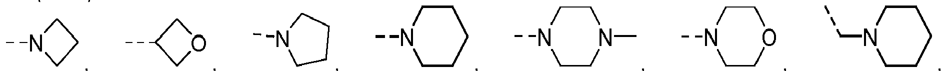 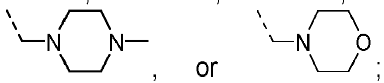
**R^{N}** represents -H, -CH₃, -C₂H₅, -C₃H₇, -CH(CH₃)₂, -C₄H₉, -CH₂-CH(CH₃)₂, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -Ph, -CH₂Ph, -COCH₃, -COCF₃, -COC₂H₅, -COC₃H₇, -COCH(CH₃)₂, -COC₄H₉, -COCH₂-CH(CH₃)₂, -COC(CH₃)₃, -CO-cyclo-C₃H₅, -CO-cyclo-C₄H₇, -CO-cyclo-C₅H₉, -CO-cyclo-C₆H₁₁, -COPh, -COCH₂Ph, -CO₂CH₃, -CO₂CF₃, -CO₂C₂H₅, -CO₂C₃H₇, -CO₂CH(CH₃)₂, -CO₂C₄H₉, -CO₂CH₂-CH(CH₃)₂, -CO₂C(CH₃)₃, -CO₂-cyclo-C₃H₅, -CO₂-cyclo-C₄H₇, -CO₂-cyclo-C₅H₉, -CO₂-cyclo-C₆H₁₁, -CO₂Ph, -CO₂CH₂Ph, -SO₂CH₃, -SO₂CF₃, -SO₂C₂H₅, -SO₂C₃H₇, -SO₂CH(CH₃)₂, -SO₂C₄H₉, -SO₂CH₂-CH(CH₃)₂, -SO₂C(CH₃)₃, -SO₂-cyclo-C₃H₅, -SO₂-cyclo-C₄H₇, -SO₂-cyclo-C₅H₉, -SO₂-cyclo-C₆H₁₁, -SO₂Ph, or -SO₂CH₂Ph;
or an enantiomer, a stereoisomeric form, a mixture of enantiomers, a diastereomer, a mixture of diastereomers, a hydrate, a solvate, a racemate of the above-mentioned compounds, or a pharmaceutically acceptable salt thereof.

2. The compound according to Claim 1 represented by one of the formulae (**Ia**) - (**Id**), and (**IIa**) - (**IId**): 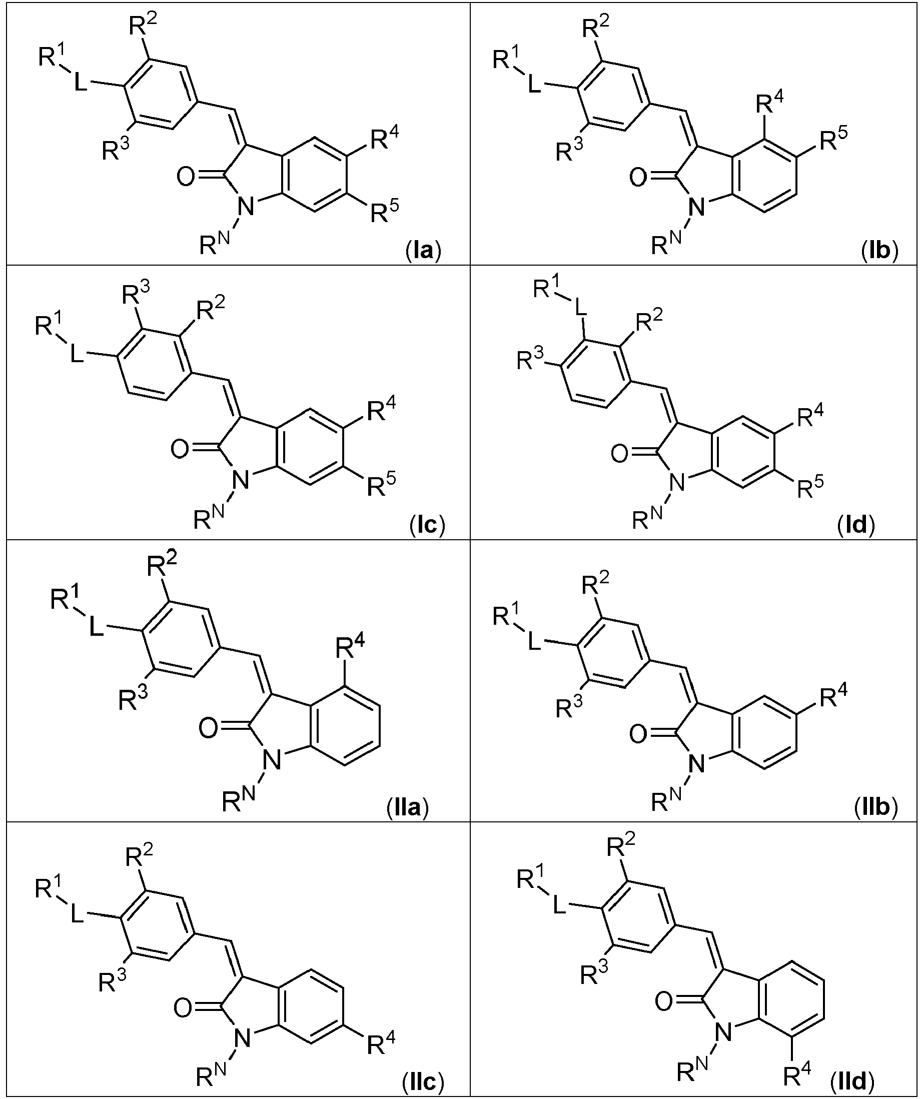 wherein **L**, **R¹**, **R²**, **R³**, **R⁴**, **R⁵** and **R^{N}** have the same meanings as defined in Claim 1.

3. The compound according to Claim 1 or 2, wherein
L represents **-L₃-L₂-L₁-**;
**L₁** represents a covalent bond or -NH-CO-CH₂-O-;
**L₂** represents -(C₂H₄O)ₙ- or -(CH₂)ₘ-(OC₂H₄)ₙ-,
**L₃** represents -CO-NH- or -CH₂-CO-NH-;
**n** is an integer selected from 1, 2, 3, 4, and 5;
**m** is an integer selected from 1 or 2;
**R²** and **R³** represent independently of each other -F, -Cl, or -Br;
**R⁴** represents -F, -Cl, -Br, or -I; and
**R^{N}** represents -H, -CH₃, or -Ph.

4. The compound according to any one of the Claims 1 to 3, wherein **R¹** represents

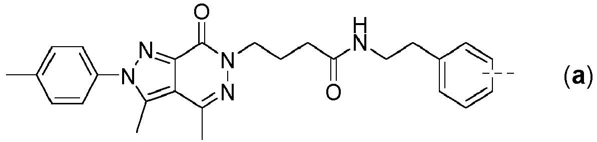

5. The compound according to any one of the Claims 1 to 3, wherein **R¹** represents

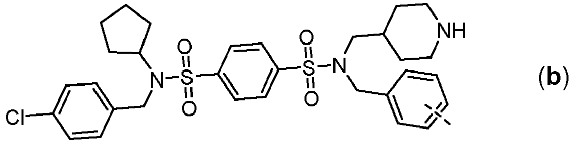

6. The compound according to any one of the Claims 1 to 3, wherein **R¹** represents

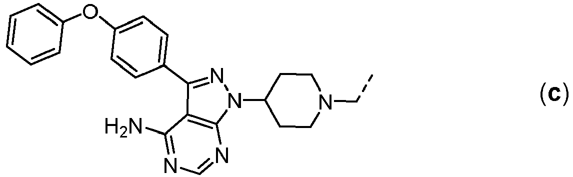

7. The compound according to claim 1 selected from the group consisting of:
Comp. **5:** (Z)-N-(2-(2-(2-(2-(2,6-dibromo-4-((5-bromo-2-oxoindolin-3-ylidene)methyl)phenoxy) ethoxy)ethoxy)ethoxy)ethyl)-4-(2-(4-(3,4-dimethyl-7-oxo-2-(p-tolyl)-2,7-dihydro-6H-pyrazolo[3,4-d] pyridazin-6-yl)butanamido)ethyl)benzamide (**5**) 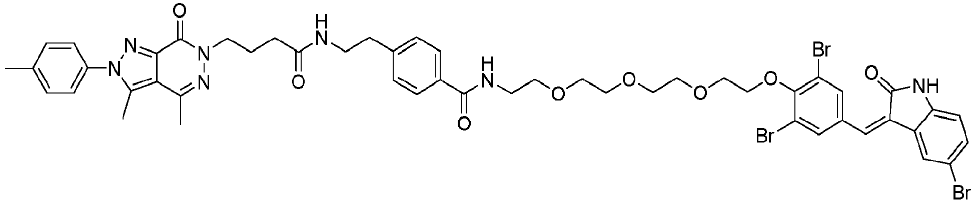
Comp. **6:** (Z)-N-(2-(2-(2-(2-(2,6-dibromo-4-((5-iodo-2-oxoindolin-3-ylidene)methyl)phenoxy) ethoxy)ethoxy)ethoxy)ethyl)-4-(2-(4-(3,4-dimethyl-7-oxo-2-(p-tolyl)-2,7-dihydro-6H-pyrazolo[3,4-d]pyridazin-6-yl)butanamido)ethyl)benzamide (**6**) 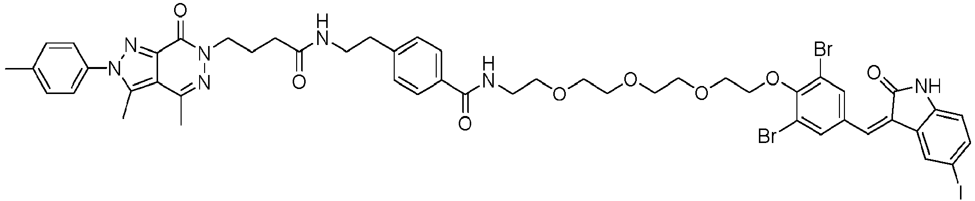
Comp. **7:** (Z)-4-(((4-(N-(4-chlorobenzyl)-N-cyclopentylsulfamoyl)-N-(piperidin-4-ylmethyl) phenyl)sulfonamido)methyl)-N-(2-(2-(2-(2-(2,6-dibromo-4-((5-bromo-2-oxoindolin-3-ylidene)methyl)phenoxy)ethoxy)ethoxy)ethoxy)ethyl)benzamide (**7**) 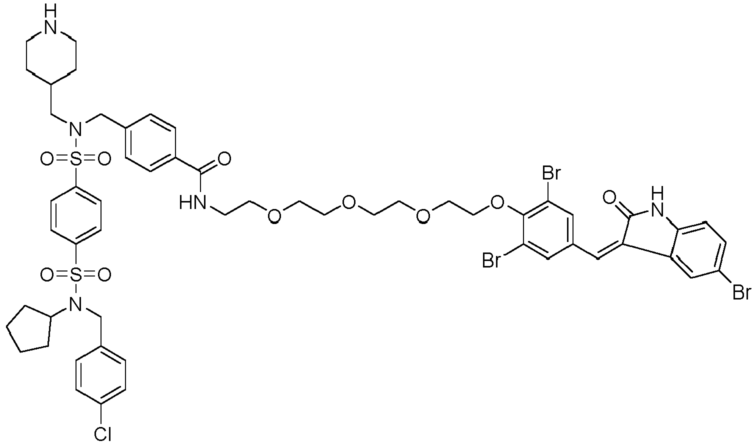
Comp. **13:** (*Z*)-2-(4-(4-amino-3-(4-phenoxyphenyl)-1H-pyrazolo[3,4-d]pyrimidin-1-yl)piperidin-1-yl)-N-(1-(2,6-dibromo-4-((5-bromo-2-oxoindolin-3-ylidene)methyl)phenoxy)-2-oxo-6,9,12,15-tetr aoxa-3-azaheptadecan-17-yl)acetamide (**13**) 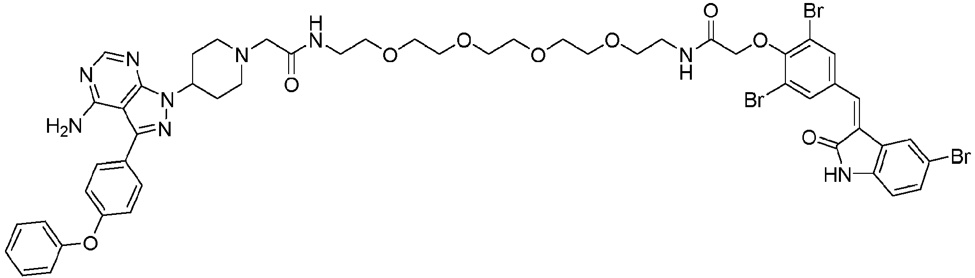 or an enantiomer, a stereoisomeric form, a mixture of enantiomers, a diastereomer, a mixture of diastereomers, a hydrate, a solvate, a racemate of the above mentioned compounds, or a pharmaceutically acceptable salts thereof.

8. A pharmaceutical composition comprising at least one compound according to any one of the claims 1 to 7 and at least one pharmaceutically acceptable carrier, excipient and/or diluent.

9. The compound according to any one of the claims 1 - 7 for use as a medicament.

10. The compound according to any one of the claims 1 - 7, or a pharmaceutically acceptable salt thereof for use, or a pharmaceutical composition according to claim 8 for use in the treatment or prophylaxis of a cancer, a tumor, an autoimmune disease, or an inflammatory disease.

11. The compound for use according to claim 9 or 10, or the pharmaceutical composition for use according to claim 10, wherein the cancer is associated with and/or caused by PDEdelta (PDEδ) / K-Ras kinase or B lymphoid *kinase (*BLK) /Bruton's tyrosine kinase (BTK); and/or
the autoimmune disease is associated with or caused by B lymphoid *kinase (*BLK) / Bruton's tyrosine kinase (BTK).

12. The compound for use according to claim 9, 10 or 11, or the pharmaceutical composition for use according to claim 10 or 11, wherein the cancer or the tumor is selected from the group comprising or consisting of:
colorectal cancer, pancreatic cancer, lung cancer, non-small lung cancer,
prostate adenocarcinoma (PRAD), bladder adenocarcinoma (BLCA), and lung squamous (LUSC) tumors, non-Hodgkin's lymphoma (NHL), *Mantle cell lymphoma* (MCL), chronic lymphocytic leukemia (CLL), lymphoplasmacytic lymphoma, Waldenström macroglobulinemia, small lymphocytic lymphoma, cutaneous T-cell lymphoma (CTCL), acute lymphoblastic leukemia, multiple myeloma, and marginal zone lymphoma.

13. The compound for use according to claim 9, 10 or 11, or the pharmaceutical composition for use according to claim 10 or 11, wherein the autoimmune disease is selected from the group comprising or consisting of: *rheumatoid arthritis (RA),* multiple sclerosis (MS), systemic lupus erythematosus (SLE), Sjogren's syndrome (SS), other B cell-associated autoimmune disorders; autoimmune haematologic conditions, primarily immune thrombocytopenia (ITP).

14. A compound of the formula (**I***) as an intermediate for the synthesis of a compound of the formula (**I**) 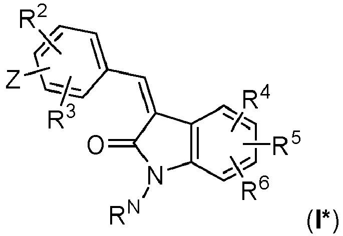 wherein
***Z*** represents -OH, -O-C₂H₄-OH, -O-CH₂-COO**R'**, or **X-L₂-L₁**-;
**L₁** represents a covalent bond, -O-, -NH-, or -NH-CO-CH₂-O-;
**L₂** represents -(C₂H₄)ₙ-, -CH₂-(C₂H₄)ₙ-, -(C₂H₄O)ₙ-, -(CH₂)ₘ-(C₂H₄O)ₙ-, -(C₂H₄O)ₙ-(CH₂)ₘ-, -(CH₂)ₘ-(OC₂H₄)ₙ-, -(OC₂H₄)ₙ-(CH₂)ₘ-, -(CH₂)ₘ-(C₂H₄O)ₙ-(CH₂)ₒ-, -(CH₂)ₘ-(OC₂H₄)ₙ-(CH₂)ₒ- or -(CH₂)ₘ-(OC₂H₄)ₙ-O-(CH₂)ₒ-;
**X** represents -OH, -NH₂, -NHCO₂-C(CH₃)₃, -CO₂**R'**, -Br, -Cl, -I, or -OSO₂CH₃;
**R'** represents -H, -CH₃, -C₂H₅, -CH₂CH(CH₃)₂, or -C(CH₃)₃,
**n** is an integer selected from 1, 2, 3, 4, 5, 6, 7, 8, 9, and 10;
**m** and **o** are independently of each other an integer selected from 1, 2, 3, and 4;
**R²** and **R³** represent -Br;
**R⁴, R⁵, R⁶**, and **R^{N}** have the same meanings as defined in Claim 1,
and two of **R⁴, R⁵,** and **R⁶** are not -CI at the same time;
or an enantiomer, a stereoisomeric form, a mixture of enantiomers, a diastereomer, a mixture of diastereomers, a hydrate, a solvate, a racemate of the above mentioned compounds, or a salt thereof.

15. The compound according to claim 14, selected from the group consisting of compounds **2**, **39** - **46**, **57**, **58**, **61**, and **62**:
